# EUROPEAN PATENT SPECIFICATION

(11) **EP 4 619 409 B1**
(45) Date of publication and mention of the grant of the patent: **04.11.2026**
(21) Application number: 23805600.6
(22) Date of filing: 14.11.2023
(51) Int. Cl.: C07H 1/00, C07H 9/04

(54) **METHOD FOR PREPARING AN AT LEAST PARTIALLY ACETAL-PROTECTED SUGAR**
VERFAHREN ZUR HERSTELLUNG EINES ZUMINDEST TEILWEISE ACETAL-GESCHÜTZTEN ZUCKERS
PROCÉDÉ DE PRÉPARATION D'UN SUCRE AU MOINS PARTIELLEMENT PROTÉGÉ PAR DES ACÉTALS

(30) Priority: 15.11.2022 EP 22207430
(43) Date of publication of application: 24.09.2025
(73) Proprietor: ECOLE POLYTECHNIQUE FEDERALE DE LAUSANNE (EPFL), 1015 Lausanne (CH)
(72) Inventor: LI, Zezhong, 1008 Prilly (CH); NELIS, Tom, 1110 Morges (CH); MANKER, Lorenz, 1006 Lausanne (CH); SUN, Songlan, 1024 Ecublens (CH); KOMAROVA, Anastasiia, 1022 Chavannes-Renens (CH); LUTERBACHER, Jeremy, 1022 Chavannes-Renens (CH)
(74) Representative: Schaad, Balass, Menzl & Partner AG
(86) International application number: PCT/EP2023/081790
(87) International publication number: WO 2024/105051

(56) References cited:
- EP-A1- 3 170 830
- WO-A1-2010/115934
- KONGKATHIP BOONSONG ET AL: "New Strategy for Synthesis of the Disaccharide Moiety of the Highly Potent Anticancer Natural Product OSW-1", SYNTHETIC COMMUNICATIONS, vol. 44, no. 15, 3 August 2014 (2014-08-03), US, pages 2248 - 2255, XP093039408, ISSN: 0039-7911, DOI: 10.1080/00397911.2014.891747
- MIETHCHEN RALF ET AL: "New Effective Transformations of Monosaccharides by Selective Inversion", LIEBIGS ANNALEN DER CHEMIE, vol. 1994, no. 12, 30 November 1994 (1994-11-30), DE, pages 1191 - 1197, XP093039381, ISSN: 0170-2041, DOI: 10.1002/jlac.199419941210
- MATSUZAWA MASAFUMI ET AL: "Branched-chain Sugars. XXII. Synthesis of Benzyl 2,3-Di- O -benzyl- and 2,3- O -Methylene-[beta]-L- threo -pentopyranosid-4-uloses, and the Corresponding 6-Deoxy-[alpha]-D- xylo -hexopyranosid-4-uloses", BULLETIN OF THE CHEMICAL SOCIETY OF JAPAN, vol. 54, no. 7, 1 July 1981 (1981-07-01), JP, pages 2169 - 2173, XP093039390, ISSN: 0009-2673, DOI: 10.1246/bcsj.54.2169
- LIPTÁK ANDRÁS ET AL: "Synthesis and hydrogenolysis of the methylene, ethylidene, isopropylidene, and diastereoisomeric 1-phenylethylidene acetals of [beta]-l-arabino- and [alpha]-l-rhamnopyranoside derivatives", CARBOHYDRATE RESEARCH, vol. 138, no. 1, 1 April 1985 (1985-04-01), GB, pages 1 - 15, XP093039485, ISSN: 0008-6215, DOI: 10.1016/0008-6215(85)85218-6

## Description

The present invention relates to a method for preparing an at least partially acetal-protected sugar.

In recent decades, the gradual depletion of fossil fuel resources, increase in global energy consumption, and environmental issues have driven the development of new materials and technologies that utilize renewable biological sources such as biomass or food waste. One of the cutting-edge topics in this area is the development of bio-based chemicals that are considered as greener alternatives to petroleum-derived chemicals. In principle, bio-based chemicals often do not result in a net increase of carbon dioxide in the atmosphere at the end of their lifetimes and thus ensure less environmental damages.

WO2022/223480 discloses acetal-protected xylose-diformylxylose (DFX) and its derivatives as green polar aprotic solvents. Example 1 demonstrates a synthesis route from commercial D-xylose and paraformaldehyde using a homogeneous acidic catalyst (H₂SO₄) which has to be added drop-wise in order to avoid the degradation of the sugar.

WO2021074211 discloses a method for preparing polymerizable monomers from renewable resources such as biomass. It was shown that glyoxylic acid protected xylose can be produced in the presence of a homogenous acidic catalyst. Said glyoxylic acid protected xylose can be used in polymer synthesis.

Y. M. Questell-Santiago, R. Zambrano-Varela, M. Talebi Amiri and J. S. Luterbacher, Nat. Chem. 2018, 10, 1222-1228 disclosed that DFX can be directly synthesized from D-Xylose in the presence of 37 wt% aqueous solution of formaldehyde and 37 wt% aqueous solution of HCl using 1,4 dioxane as solvent and n-hexane as extraction solvent.

WO 2010/115934 A1 discloses the synthesis of a trisaccharide, while EP 3170830 A1 describes the synthesis of 5-azido-5-deoxy-2:3-isopropylidene D-arabinose, namely the hydrolysis of small saccharide derivatives.

Kongkathip Boonsong et al. ("New StratSynthetic Communications, vol. 44, no. 15, 3 August 2014, pp. 2248-2255) disclose the synthesis of a partially protected OSW-1 disaccharide moiety. This compound, featuring a 2-O-p-methoxybenzoyl-β-p-xylopyranosyl-(1→3)-2-O-acetyl-L-arabinopyranoside structure, was elaborated through β-stereoselective glycosylation.

Miethchen Ralf et al. ("New Effective Transformations of Monosaccharides by Selective Inversion," Liebigs Annalen der Chemie, vol. 1994, no. 12, 30 November 1994, pp. 1191-1197) disclose regio- and stereoselective acetalizations of methyl α-D-lyxo-pyranoside, methyl β-L-arabinopyranoside, methyl α- and β-galactopyranoside, and methyl β-D-galactopyranoside with chloral, including inversion of configuration at C-3, using dicyclohexylcarbodiimide as a coagent.

Matsuzawa Masafumi et al. ("Branched-chain Sugars. XXII. Synthesis of Benzyl 2,3-Di-O-benzyl- and 2,3-O-methylene-β-L-threo-pentopyranosid-4-uloses, and the Corresponding 6-Deoxy-α-D-xylo-hexopyranosid-4-uloses," Bulletin of the Chemical Society of Japan, vol. 54, no. 7, 1 July 1981, pp. 2169-2173) disclose the synthesis of benzyl 2,3-di-O-benzyl- and 2,3-O-methylene-β-L-threo-pentopyranosid-4-uloses, and the corresponding 6-deoxy-α-D-xylo-hexopyranosid-4-uloses from L-arabinose and D-glucose.

Lipták András et al. ("Synthesis and hydrogenolysis of the methylene, ethylidene, isopropylidene, and diastereoisomeric 1-phenylethylidene acetals of β-L-arabino- and α-L-rhamnopyranoside derivatives," Carbohydrate Research, vol. 138, no. 1, 1 April 1985, pp. 1-15) disclose the synthesis and hydrogenolysis of the methylene, ethylidene, isopropylidene, and diastereoisomeric 1-phenylethylidene acetals of β-L-arabino- and α-L-rhamnopyranoside derivatives. All methods require the constant addition and neutralization of a homogenous acid, which is not favourable in large scale processing for the additional cost and waste management.

The object of the present invention was to provide a simpler method for preparing acetal-protected sugars in high yields using scalable methods.

The problem is solved by the method according to claim 1. Further preferred embodiments are subject of dependent claims 2 to 15.

It was found that an at least partially acetal-protected sugar can be prepared with a method involving the step of reacting a sugar or a sugar derivative selected from the group consisting of an aldopentose, an aldohexose, an aldopentoside and an aldohexoside with an aldehyde or an aldehyde source in the presence of a heterogeneous acidic catalyst to form the at least partially acetal-protected sugar selected from the group consisting of a compound of formula I, II, III, IV, V, VI, VII, VIII, IX, X, XI and XII 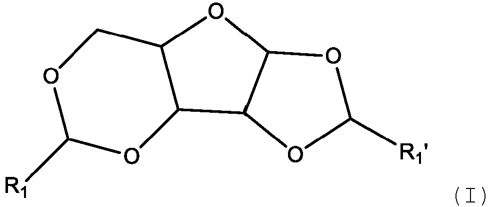 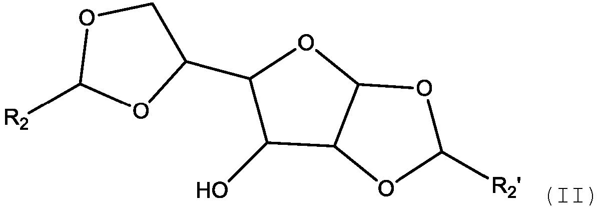 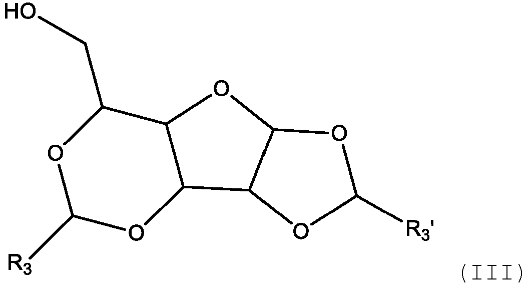 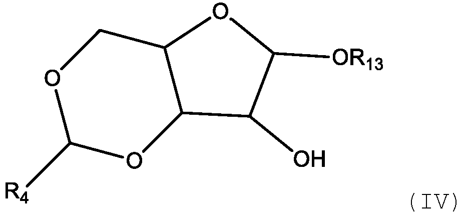 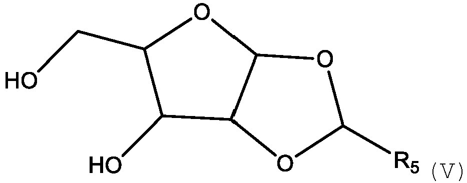 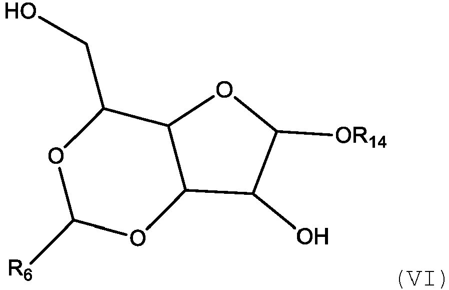 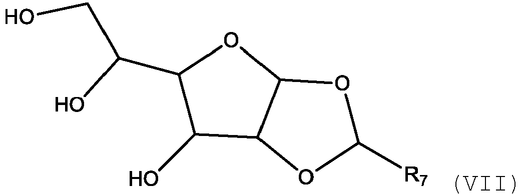 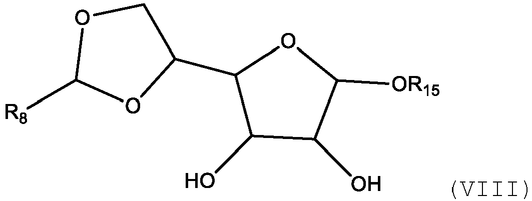 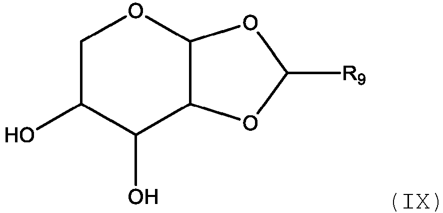 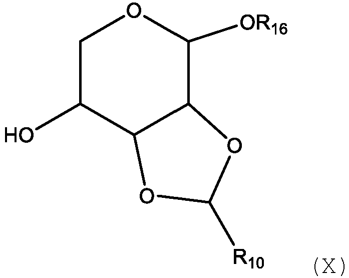 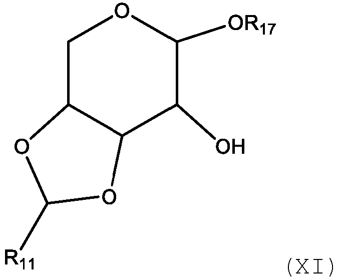 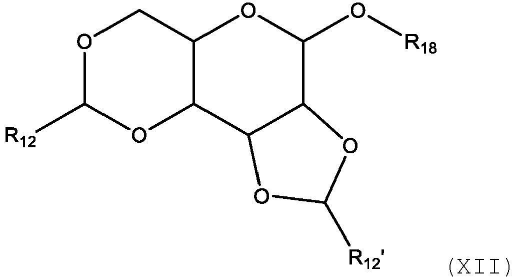
wherein R₁, R₁', R₂, R₂', R₃, R₃', R₄, R₅, R₆, R₇, R₈, R₉, R₁₀, R₁₁, R₁₂, and R₁₂' are Y or Z-E, and wherein R₁ and R₁', R₂ and R₂', R₃ and R₃', and R₁₂ and R₁₂' are the same or different from each other and
Y is hydrogen or a linear, branched or cyclic hydrocarbon moiety having 1 to 20 carbon atoms,
Z is a linear, branched or cyclic hydrocarbon moiety with 0 to 12 carbon atoms, optionally substituted with 1 to 4 C₁ to C₄ alkyl groups, 1 to 4 halogen atoms, or benzyl groups and
E is -COOH, -CH(COOH)₂, -COOR₁₉, -CH(COOR₂₀)(COOR₂₁), -CHO, -CH(CHO)₂, -C₂H₃, -CH(C₂H₃) ₂, -CHCHR₂₂, -CHCR₂₃R₂₄, -C₂H, -C₂R₂₅, -N₃, -NH₂, -CH(NH₂)₂, -NHR₂₆, -CH(NHR₂₇)(NHR₂₈), -NR₂₉R₃₀, -CH(NR₃₁R₃₂)(NR₃₃R₃₄), -OH, -OR₃₅, -CH(R₃₆OH)(R₃₇OH), and
R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, and R₃₅, are independent from each other C₁ to C₂₀ alkyl, and
R₂₀ and R₂₁, R₂₃ and R₂₄, R₂₇ and R₂₈, R₂₉ and R₃₀, R₃₁ and R₃₂, as well as R₃₃ and R₃₄ are the same or different from each other, and
R₃₆ and R₃₇ are independent from each other absent or a linear or branched C₁ to C₁₂hydrocarbon chain and
R₁₃, R₁₄, R₁₅, R₁₆, R₁₇ and R₁₈ are independent from each other hydrogen or a linear, branched or cyclic hydrocarbon moiety having 1 to 20 carbon atoms.

By using a heterogenous acidic catalyst, the need of constant addition and neutralization of a homogeneous acid is alleviated. Said catalyst can be easily recycled for example by filtration, which is economically and ecologically advantageous. In addition, the catalyst can be used as the packing material in a continuous flow reactor for large-scale production. The yield and selectivity of acetal-protected sugars can also be improved as compared to the traditional homogeneous catalyst systems, such as with HCl or H₂SO₄.

Moreover, despite the high reactivity of aldehydes towards undesired aldol condensation, oxidation, self-polymerization and formation of geminal diols during reactions, it is possible to obtain compounds of formula I to XII in the presence of a heterogenous catalyst. Furthermore, the unique hydrate formation of aldehydes allows the rate and selectivity of sugar protection to be controlled by tuning the water content in the reaction system.

The selectivity of acetal protected sugars can be further enhanced by the heterogeneous catalysts of various pore dimensions and affinities to reactants and products. The selectivity towards small products will be enhanced with heterogeneous catalysts of small pores, e.g., the selective formation of compounds of formula IV to XI over compounds of formula I, II, III, and XII, or the selective formation of compounds of formula I to XII with smaller R groups (selected from R₁, R₁', R₂, R₂', R₃, R₃', R₄, R₅, R₆, R₇, R₈, R₉, R₁₀, R₁₁, R₁₂, and R₁₂' )over bulkier R groups (selected from R₁, R₁', R₂, R₂', R₃, R₃', R₄, R₅, R₆, R₇, R₈, R₉, R₁₀, R₁₁, R₁₂, and R₁₂'). On the contrary, the selectivity towards large products will be enhanced with heterogeneous catalysts of large pores, e.g., the selective formation of compounds of formula I, II, III, and XII over compounds of formula IV to XI, or the selective formation of compounds of formula I to XII with bulkier R groups over smaller R groups. Similarly, the selectivity can be tuned with the affinity between the aldehyde and the heterogeneous catalyst. High affinity between the aldehyde and the heterogeneous catalyst leads to the high selectivity towards the formation of compounds of formula I, II, III, and XII over the compounds of formula IV to XI. Low affinity between the aldehyde and the heterogeneous catalyst leads to the high selectivity towards the formation of compounds of formula IV to XI over the compounds of formula I, II, III, and XII.

Some of the compounds produced by the method according to the present invention can be used as green polar aprotic solvents. Of those that can be used as these types of solvents, some of them have comparable or better performance compared to their conventional fossil-based analogues. Moreover, they have many other applications. For example, they can be used as platform molecules for the production of many other bio-based products therefore, their efficient production is important. For example, DFX can be a starting compound to produce the food additive xylitol. Partially acetal-protected sugars with long chain hydrocarbon aldehydes, such as alkyl or alkenyl aldehydes, may also be used as biobased surfactants. Fully protected sugars with ester, carboxylic or hydroxyl in their corresponding R groups can be used as building blocks for polymer production.

Within the context of the present invention the term "aldopentose" means a pentose with an aldehyde functional group as the endmost carbon atom and is preferably selected from the group consisting of D-ribose, L-ribose, D-arabinose, L-arabinose, D-xylose, L-xylose, D-lyxose and L-lysose. Due to sugar tautomerization, the said aldopentose can be used in linear, pyranose or furanose conformations or a mixture thereof.

Within the context of the present invention the term "aldohexose" means a hexose with an aldehyde functional group as the endmost carbon atom and is preferably selected from the group consisting of D-allose, L-allose, D-altrose, L-altrose, D-glucose, L-glucose, D-mannose, L-mannose, D-gulose, L-gulose, D-idose, L-idose, D-galactose, L-galactose, D-talose, and L-talose. Due to sugar tautomerization, the said aldohexose can be used in linear, pyranose or furanose conformations or a mixture thereof.

Within the context of the present invention the term "aldopentoside" means a pentose as defined above bound to a functional group selected from the group consisting of a linear, branched or cyclic hydrocarbon moiety having 1 to 20 carbon atoms, preferably a C₁ to C₄ alkyl group, at the oxygen atom in position 1 of the aldopentose by a glycosidic bond, thus for a 1-O-alkylaldopentose. It is preferably selected from the group consisting of 1-O-alkyl-D-ribose, 1-O-alkyl-L-ribose, 1-O-alkyl-D-arabinose, 1-O-alkyl-L-arabinose, 1-O-alkyl-D-xylose, 1-O-alkyl-L-xylose, 1-O-alkyl-D-lyxose and 1-O-alkyl-L-lysose, and the alkyl group is preferably selected from the group consisting of methyl, ethyl, propyl and butyl, most preferably methyl. Most preferably, the aldopentoside is 1-O-methyl-D-xylose. Due to sugar tautomerization, the said alkylaldopentose can be used in linear, pyranose or furanose conformations or a mixture thereof. Due to the additional protection of the hydroxyl group in position 1 of the sugar, said starting material could lead to more facile production of single-sided acetal monomers containing a free hydroxyl group. This could lead to bifunctional monomers (such as hydroxyacids, if glyoxylic acid were used).

Within the context of the present invention the term "aldohexoside" means a hexose as defined above bound to a functional group selected from the group consisting of a linear, branched or cyclic hydrocarbon moiety having 1 to 20 carbon atoms, preferably a C₁ to C₄ alkyl group, at the oxygen atom in position 1 of the aldohexose by a glycosidic bond, thus for a 1-O-alkylaldohexose. It is preferably selected from the group consisting of 1-O-alkyl-D-allose, 1-O-alkyl-L-allose, 1-O-alkyl-D-altrose, 1-O-alkyl-L-altrose, 1-O-alkyl-D-glucose, 1-O-alkyl-L-glucose, 1-O-alkyl-D-mannose, 1-O-alkyl-L-mannose, 1-O-alkyl-D-gulose, 1-O-alkyl-L-gulose, 1-O-alkyl-D-idose, 1-O-alkyl-L-idose, 1-O-alkyl-D-galactose, 1-O-alkyl-L-galactose, 1-O-alkyl-D-talose, and 1-O-alkyl-L-talose, and the alkyl group is preferably selected from the group consisting of methyl, ethyl, propyl, and butyl, most preferably methyl. Most preferably, the aldohexoside is 1-O-methyl-D-glucose. Due to sugar tautomerization, the said alkylaldohexose can be used in linear, pyranose or furanose conformations or a mixture thereof. Due to the additional protection of the hydroxyl group in position 1 of the sugar, said starting material leads to a more stable product without free -OH groups, which can be useful to produce linear polymers.

The term "at least partially acetal-protected sugar" stands for a sugar or a sugar derivative selected from the group consisting of an aldopentose, an aldohexose, an aldopentoside and an aldohexoside that is either partially protected with an aldehyde, meaning that only two hydroxyl groups form together with an aldehyde a cyclic acetal (thus forming a compound of formula IV, V, VI, VII, VIII, IX, X or XI) or the aldopentose, aldohexose or aldohexoside is fully protected, meaning that four hydroxyl groups form together with two aldehydes two corresponding cyclic acetals (thus forming compounds I, II, III or XII).

An aldehyde is an organic compound containing the group -CHO and an aldehyde source is a polymeric or oligomeric compound that can generate an aldehyde in the reaction conditions. The term aldehyde also encompasses the geminal diol of the corresponding aldehyde, i.e. aldehyde hydrates.

One embodiment of the present invention relates to the production of fully protected compounds selected from the group consisting of compounds I, II, III and VII:

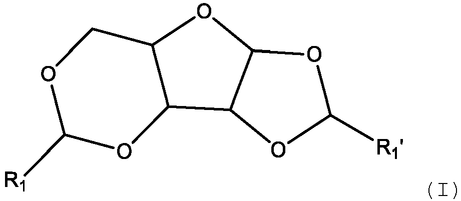

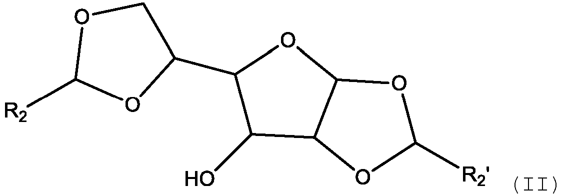

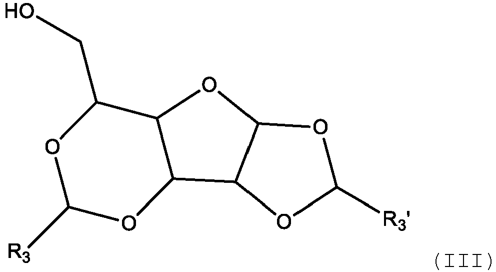

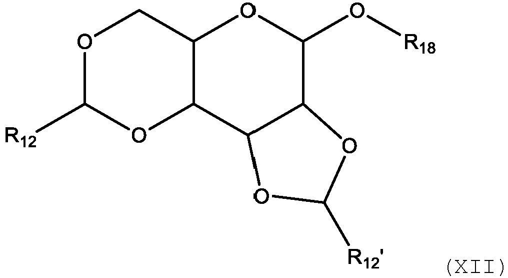

In one embodiment of the present invention R₁₃, R₁₄, R₁₅, R₁₆, and R₁₇ are hydrogen resulting in an at least partially protected compound selected from the group consisting of compound IVa, VIa, VIIIa, Xa and XIa:

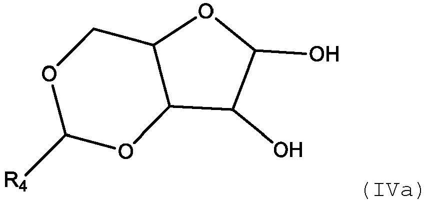

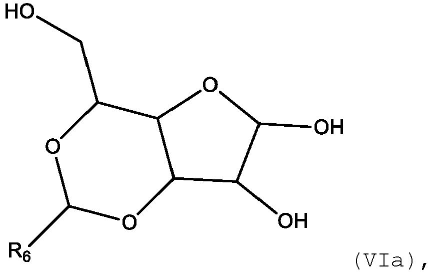

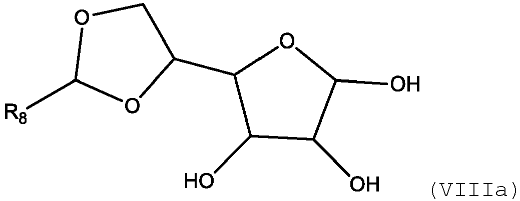

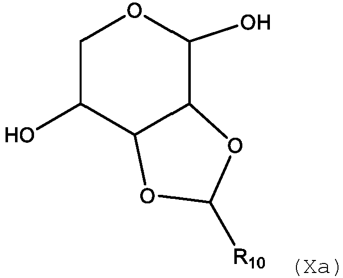

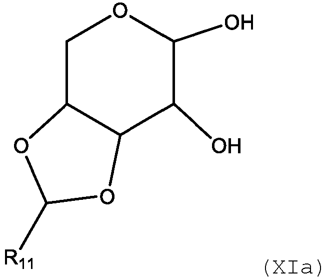

In one embodiment of the present invention R₁₃, R₁₄, R₁₅, R₁₆, and R₁₇ are a linear, branched or cyclic hydrocarbon moiety having 1 to 20 carbon atoms, preferably 1 to 6 carbon atoms, most preferably methyl, ethyl, propyl and butyl, and ideally methyl resulting in an at least partially protected compound selected from the group consisting of compound IVb, VIb, VIIIb, Xb, XIb and XII:

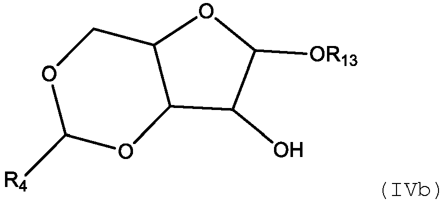

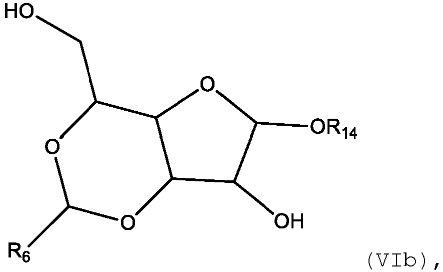

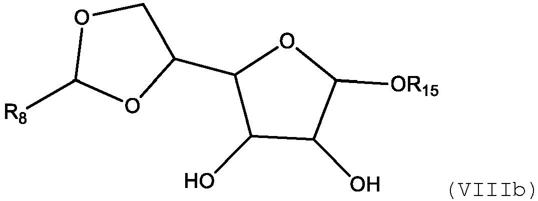

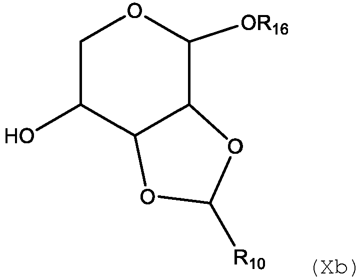

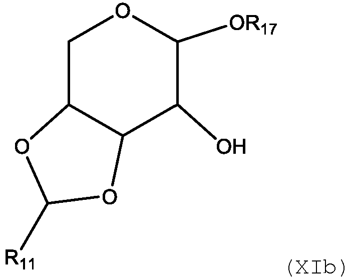

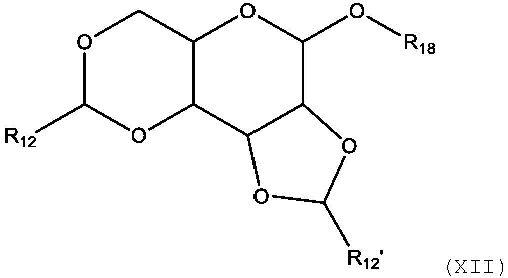

In one embodiment of the present invention R₁, R₁', R₂, R₂', R₃, R₃', R₄, R₅, R₆, R₇, R₈, R₉, R₁₀, R₁₁, R₁₂, and R₁₂' in compounds I to XII is Y and Y is hydrogen.

In a further embodiment of the present invention R₁, R₁', R₂, R₂', R₃, R₃', R₄, R₅, R₆, R₇, R₈, R₉, R₁₀, R₁₁, R₁₂, and R₁₂' in compounds I to XII is Y, and is selected from the group consisting of a linear or branched C1 to C₂₀ alkyl, a linear or branched C₂ to C₂₀ alkenyl, a cyclic aliphatic ring or aromatic system, preferably C₆ to C₂₀ alkyl, and a combination thereof.

The term a "linear or branched C₁ to C₂₀ alkyl" refers to a straight or branched hydrocarbon group containing 1 to 20 carbon atoms. Examples of "alkyl" as used herein include methyl, ethyl, n-propyl, isopropyl, n-butyl, isobutyl, sec-butyl and tert-butyl, pentyl, hexyl, heptyl, octyl, nonyl, decyl, undecyl, dodecyl, tridecyl, tetradecyl, pentadecyl, hexadecyl, heptadecyl, octadecyl, nonadecyl and eicosyl.

The term "a linear or branched C₂ to C ₂₀ alkenyl" refers to a straight or branched hydrocarbon group containing 2 to 20 carbon atoms and having at least one carbon-carbon double bond. Examples of "alkenyl", as used herein include, vinyl (ethenyl), propenyl, 2-methyl-1-propenyl, 1- butenyl, 2-butenyl, and isobutenyl.

The term "cyclic aliphatic ring or aromatic system" refers an aliphatic or aromatic ring system with 3 to 10, preferably 5 to 8 , carbon atoms.

In one embodiment of the present invention R₁, R₁', R₂, R₂', R₃, R₃', R₄, R₅, R₆, R₇, R₈, R₉, R₁₀, R₁₁, R₁₂, and R₁₂' in compounds I, II, III, IV (IVa and IVb), V, VI (VIa and VIb), VII, VIII (VIIIa and VIIIb), IX, X (Xa and Xb), XI (XIa and XIb) and XII are Z-E, wherein
Z is a linear, branched or cyclic hydrocarbon moiety with 0 to 12 carbon atoms, optionally substituted with 1 to 4 C₁ to C₄ alkyl groups, 1 to 4 halogen atoms, or benzyl groups and
E is -COOH, -CH(COOH)₂, -COOR₁₉, -CH(COOR₂₀)(COOR₂₁), -CHO, -CH(CHO)₂, - C₂H₃, CH(C₂H₃)₂, -CHCHR₂₂, -CHCR₂₃R₂₄, -C₂H, -C₂R₂₅, -N₃, -NH₂, -CH(NH₂)₂, -NHR₂₆, -CH(NHR₂₇)(NHR₂₈), -NR₂₉R₃₀, -CH(NR₃₁R₃₂)(NR₃₃R₃₄), -OH, -OR₃₅, - CH(R₃₆OH)(R₃₇OH), and
R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, and R₃₅, are independent from each other C₁ to C₂₀ alkyl, and
R₂₀ and R₂₁, R₂₃ and R₂₄, R₂₇ and R₂₈, R₂₉ and R₃₀, R₃₁ and R₃₂, as well as R₃₃ and R₃₄ are the same or different from each other, and
R₃₆ and R₃₇ are independent from each other absent or a linear or branched C₁ to C₁₂hydrocarbon chain and
R₁₃, R₁₄, R₁₅, R₁₆, R₁₇ and R₁₈ are independent from each other hydrogen or a linear, branched or cyclic hydrocarbon moiety having 1 to 20 carbon atoms.

According to an embodiment of the invention, in the I, II, III, IV (IVa and IVb), V, VI (VIa and VIb), VII, VIII (VIIIa and VIIIb), IX, X (Xa and Xb), XI (XIa and XIb) and XII, Z-E is preferably
-(CH₂)ₘCOOH; -C₆H₄COOH, wherein the aromatic ring is optionally substituted with 1 to 4 C₁-C₄-alkyl groups or 1 to 4 halogen atoms; - C₆H₁₀COOH, wherein the aliphatic ring is optionally substituted with 1 to 10 C₁-C₄-alkyl groups; -(CH₂)ₘCH(COOH)₂;
-(CH₂)ₘCOOR₁₉; -C₆H₄COOR₁₉, wherein the aromatic ring is optionally substituted with 1 to 4 C₁-C₄-alkyl groups or 1 to 4 halogen atoms; - C₆H₁₀COOR₁₉, wherein the aliphatic ring is optionally substituted with 1 to 10 C₁-C₄-alkyl groups;
-(CH₂)ₘCH(COOR₂₀)(COOR₂₁); -C₆H₄CH(COOR₂₀)(COOR₂₁), wherein the aromatic ring is optionally substituted with 1 to 4 C₁-C₄-alkyl groups or 1 to 4 halogen atoms; -C₆H₁₀CH(COOR₂₀)(COOR₂₁), wherein the aliphatic ring is optionally substituted with 1 to 10 C₁-C₄-alkyl groups;
-(CH₂)ₘCHO; -C₆H₄CHO, wherein the aromatic ring is optionally substituted with 1 to 4 C₁-C₄-alkyl groups or 1 to 4 halogen atoms; - C₆H₁₀CHO, wherein the aliphatic ring is optionally substituted with 1 to 10 C₁-C₄-alkyl groups or 1 to 4 halogen atoms; -(CH₂)ₘCH(CHO)₂;
-(CH₂)ₘC₂H₃; -C₆H₄C₂H₃, wherein the aromatic ring is optionally substituted with 1 to 4 C₁-C₄-alkyl groups or 1 to 4 halogen atoms; - C₆H₁₀C₂H₃, wherein the aliphatic ring is optionally substituted with 1 to 10 C₁-C₄-alkyl groups; -(CH₂)ₘCH(C₂H₃)₂;
-(CH₂)ₘCHCHR₂₂ ; -C₆H₄CHCHR₂₂ , wherein the aromatic ring is optionally substituted with 1 to 4 C₁-C₄-alkyl groups or 1 to 4 halogen atoms; - C₆H₁₀CHCHR₂₂ , wherein the aliphatic ring is optionally substituted with 1 to 10 C₁-C₄-alkyl groups;
-(CH₂)ₘCHCR₂₃R₂₄ ; -C₆H₄CHCR₂₃R₂₄, wherein the aromatic ring is optionally substituted with 1 to 4 C₁-C₄-alkyl groups or 1 to 4 halogen atoms; - C₆H₁₀CHCR₂₃R₂₄, wherein the aliphatic ring is optionally substituted with 1 to 10 C₁-C₄-alkyl groups;
-(CH₂)ₘC₂H; -C₆H₄C₂H, wherein the aromatic ring is optionally substituted with 1 to 4 C₁-C₄-alkyl groups or 1 to 4 halogen atoms; - C₆H₁₀C₂H, wherein the aliphatic ring is optionally substituted with 1 to 10 C₁-C₄-alkyl groups;
-(CH₂)ₘC₂R₂₅; -C₆H₄C₂R₂₅, wherein the aromatic ring is optionally substituted with 1 to 4 C₁-C₄-alkyl groups or 1 to 4 halogen atoms; - C₆H₁₀C₂R₂₅ , wherein the aliphatic ring is optionally substituted with 1 to 10 C₁-C₄-alkyl groups;
-(CH₂)ₘN₃; -C₆H₄N₃, wherein the aromatic ring is optionally substituted with 1 to 4 C₁-C₄-alkyl groups or 1 to 4 halogen atoms; -C₆H₁₀N₃, wherein the aliphatic ring is optionally substituted with 1 to 10 C₁-C₄-alkyl groups;
-(CH₂)ₘNH₂; -C₆H₄NH₂, wherein the aromatic ring is optionally substituted with 1 to 4 C₁-C₄-alkyl groups or 1 to 4 halogen atoms; - C₆H₁₀NH₂, wherein the aliphatic ring is optionally substituted with 1 to 10 C₁-C₄-alkyl groups; -(CH₂)ₘCH(NH₂)₂;
-(CH₂)ₘNHR₂₆ ; -C₆H₄NHR₂₆ , wherein the aromatic ring is optionally substituted with 1 to 4 C₁-C₄-alkyl groups or 1 to 4 halogen atoms; - C₆H₁₀NHR₂₆ , wherein the aliphatic ring is optionally substituted with 1 to 10 C₁-C₄-alkyl groups;
-(CH₂)ₘNR₂₉R₃₀; -C₆H₄NR₂₉R₃₀, wherein the aromatic ring is optionally substituted with 1 to 4 C₁-C₄-alkyl groups or 1 to 4 halogen atoms; - C₆H₁₀NR₂₉R₃₀, wherein the aliphatic ring is optionally substituted with 1 to 10 C₁-C₄-alkyl groups;
-(CH₂)ₘCH(NR₃₁R₃₂)(NR₃₃R₃₄); -C₆H₄ CH(NR₃₁R₃₂)(NR₃₃R₃₄), wherein the aromatic ring is optionally substituted with 1 to 4 C₁-C₄-alkyl groups or 1 to 4 halogen atoms; -C₆H₁₀CH(NR₃₁R₃₂)(NR₃₃R₃₄), wherein the aliphatic ring is optionally substituted with 1 to 4 C₁-C₄-alkyl groups;
-(CH₂)ₘOH; -C₆H₄OH, wherein the aromatic ring is optionally substituted with 1 to 4 C₁-C₄-alkyl groups or 1 to 4 halogen atoms; -C₆H₁₀OH, wherein the aliphatic ring is optionally substituted with 1 to 10 C₁-C₄-alkyl groups;
-(CH₂)ₘOR₃₅; -C₆H₄OR₃₅ , wherein the aromatic ring is optionally substituted with 1 to 4 C₁-C₄-alkyl groups or 1 to 4 halogen atoms; - C₆H₁₀OR₃₅ , wherein the aliphatic ring is optionally substituted with 1 to 10 C₁-C₄-alkyl groups;
-CH(R₃₆OH)(R₃₇OH); -C₆H₄CH(R₃₆OH)(R₃₇OH), wherein the aromatic ring is optionally substituted with 1 to 4 C₁-C₄-alkyl groups or 1 to 4 halogen atoms; -C₆H₁₀CH(R₃₆OH)(R₃₇OH), wherein the aliphatic ring is optionally substituted with 1 to 10 C₁-C₄-alkyl groups;
R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄ and R₃₅, are independent from each other C₁ to C₂₀ alkyl, and
R₂₀ and R₂₁, R₂₃ and R₂₄, R₂₇ and R₂₈, R₂₉ and R₃₀, R₃₁ and R₃₂, as well as R₃₃ and R₃₄ are the same or different from each other, preferably methyl, ethyl, propyl and butyl and
R₃₆ and R₃₇ are independent from each other or absent (for example - CH(OH)₂ or CH(OH)CH₂OH)) or a linear or branched C₁ to C₁₂hydrocarbon chain, preferably methylene, ethylene, propylene or butylene.
m is an integer from 0 to 12 , in particular from 0 to 4.

Within the context of the present invention the term "heterogeneous acidic catalyst" means an acidic catalyst which is in the solid phase, and the reactants are in the liquid phase. Preferably, the acidic catalyst is a Brønsted acidic catalyst, more preferably an anhydrous Bronsted acidic catalyst. More specifically this can be a heterogeneous acidic material and/or optionally a Brønsted acidic catalyst which can be incorporated into, onto, or covalently bound to a solid support such as resin bead, membranes, porous carbon particles, zeolite materials, and other solid carriers. A solid carrier can be for example a material which comprises carbon, silicon dioxide, titanium dioxide, zirconium oxide, aluminum oxide, or any combinations of these oxides.

A Brønsted acid is capable of donating a proton and is known to a skilled person. Brønsted acids can be for example the bridging hydroxyl groups of zeolites or Brønsted acid site-functionalized resins. Examples of such Brønsted acids which can be used to functionalize a resin are preferably selected from the group consisting of sulfuric acid, phosphoric acid and methanesulfonic acid or mixtures thereof.

Preferably, the Brønsted acidic catalyst is selected from the group consisting of
a. acidic zeolites,
b. acidic doped zeolites,
c. acid site-functionalized resins,
d. acid site-functionalized oxides,
e. acidic oxides,
f. heteropolyacids and their derivates.

The term "acid site-functionalized resin" as used herein refers to a resin or polymer that acts as a medium for ion exchange, synthesized from an organic polymer substrate. The organic polymer substrate is for example a sulfonated polystyrene divinylbenzene or a sulfonated tetrafluoroethylene- based fluoropolymer-copolymer, resulting in a strongly acidic cation exchange resin or polymer. Non- limiting examples include Amberlyst-15, Amberlyst-36, Amberlyst-XNIOIO, Amberlite (e.g., Amberlite IRC120), Dowex D 2030, Nafion NR50 and Nafion SAC13.

The term "acid site-functionalized oxide" means an oxide such as zirconia, alumina or silica that is functionalized to yield e.g. a sulphated or sulphonated material. Such solid acidic metal oxides may optionally be supported onto a carrier material.

The term "acidic oxide" means metal oxides that have a Brønsted acidity such as niobia, or alumina.

The term "zeolite" as used herein refers to both natural and synthetic microporous crystalline silicate materials (including aluminosilicates, borosilicates and aluminoborosilicates) having a definite crystalline structure as determined by X-ray diffraction. A zeolite comprises a system of channels which may be interconnected with other channel systems or cavities such as side-pockets or cages. The channel systems may be three-dimensional, two- dimensional or one-dimensional. A zeolite comprises SiO₄ and XO₄ tetrahedra, wherein X can be Al (aluminum) or B (boron). A zeolite may comprise a combination of AlO₄ and BO₄ tetrahedra. In one embodiment, X is Al, and the zeolite comprises no BO₄ tetrahedra. The SiO₄ and XO₄ tetrahedra are linked at their corners via a common oxygen atom. The Atlas of Zeolite Framework Types (C Baerlocher, LB McCusker, DH Olson, 6th ed. Elsevier, Amsterdam, 2007) in conjunction with the web-based version (http://www.iza-structure.org/databases/") is a compendium of topological and structural details about zeolite frameworks, including the types of ring structures present in the zeolite and the dimensions of the channels defined by each ring type. Proven recipes and good laboratory practice for the synthesis of zeolites can be found in the "Verified synthesis of zeolitic materials" 2nd Edition 2001. Various proven recipes for the synthesis comprising BO tetrahedra are available. For example, the synthesis and characterization of boron-based zeolites having a MFI topology has been described by Cichocki and Parasiewicz-Kaczmarska (Zeolites 1990, 10, 577-582).

Suitable zeolites for use in the process according to the present invention can comprise:
- at least two, preferably two or three, non-interconnected and parallel channel systems wherein, at least one of said channel systems comprises 8- or more-membered ring channels; and a framework Si/X₂ ratio of at least 4 as measured by NMR; or
- at least two, preferably two or three, interconnected and non-parallel channel systems wherein, at least one of said channel systems comprises 10- or more-membered ring channels; and a framework Si/X₂ ratio of at least 4 as measured by NMR; or
- three interconnected and non-parallel channel systems wherein at least two of the channel systems comprise 10-or more-membered ring channels, and a framework Si/X₂ ratio of at least 4 as measured by NMR.
wherein each X is Al or B.

As used herein, the term "channel system" refers to a system of parallel or non-parallel and crystallographically equivalent channels, wherein the channels are 8-membered ring channels or larger, for example wherein the channels are 10-membered ring channels or 12-membered ring channels. Accordingly, as used herein, the term "channel" refers to an 8-or more membered ring channel which is part of a system of parallel or non-parallel and crystallographically equivalent channels.

Suitable zeolites for use in the present process comprise 10-or more-membered ring channels, such as 12-membered ring channels (12MR), or larger. The ring size for each known zeolite framework type is provided in the Atlas of Zeolite Framework Types (C Baerlocher, LB McCusker, DH Olson, 6th ed. Elsevier, Amsterdam, 2007).

As used herein the terms "8-membered ring channels" or "8MR" refer to a channel comprising unobstructed 8-membered rings, wherein the 8-membered rings define the smallest diameter of the channel. An 8-membered ring comprises 8 T atoms, and 8 alternating oxygen atoms (forming the ring), wherein each T is Si, Al or B. As used herein the terms "10-membered ring channels" or "10MR" refers to a channel comprising unobstructed 10-membered rings, wherein the 10-membered rings define the smallest diameter of the channel. A 10-membered ring comprises 10 T atoms, and 10 alternating oxygen atoms (forming the ring), wherein each T is Si, Al or B. As used herein the terms "12-membered ring channels" or "12MR" refers to a channel comprising unobstructed 12-membered rings, wherein the 12-membered rings define the smallest diameter of the channel. A 12-membered ring comprises 12 T atoms, and 12 alternating oxygen atoms (forming the ring), wherein each T is Si, Al or B. As used herein, the term "10-or-more-membered ring channel" refers to a 10-membered ring channel or larger, and therefore comprises for example both 10-membered ring channels and 12-membered ring channels.

The framework Si/X₂ ratio may be determined via Nuclear Magnetic Resonance (NMR) measurements, more particularly 29Si and 27Al NMR. In a preferred embodiment, there is no framework B, and the Si/X₂ ratio is equal to the Si/Al₂ ratio. The determination of the Si/Al₂ ratio by NMR may be performed as described by Klinowski (Ann. Rev. Mater. Sci. 1988, 18, 189-218); or as described by G. Engelhardt and D. Michel (High-Resolution Solid-State NMR of Silicates and Zeolites. John Wiley & Sons, Chichester 1987. xiv, 485 pp). The determination of the Si/B2 ratio by NMR may be performed as discussed by D. Trong On et al. (Studies in Surface Science and Catalysis 1995, 97, 535-541; Journal of Catalysis, November 1995, Volume 157, Issue 1, Pages 235-243).

Zeolites are thermostable catalysts and can therefore be regenerated by calcination, in contrast with classic thermolabile ion-exchange resins such as Amberlyst-15.

The term "heteropolyacid" as used herein refers to a compound comprising
- a metal selected from the group consisting of tungsten, molybdenum and vanadium,
- an element from the p-block of the periodic table such as silicon, phosphorus or arsenic,
- oxygen and
- hydrogen.

Preferably, the heteropolyacid is selected from the group consisting of phosphotungstic acid, silicotungstic acid, aresentungstic acid, phosphomolybdic acid, silicomolybdic acid, arsenmolybdic acid, phosphovanadic acid, silicovanadic acid and arsenvanadic acid. In another preferred embodiment the heteropolyacid derivates are produced by common derivatization techniques in order to render them solidified/immobilized, and include in particular their salts and immobilized forms that are on a solid support or resin bead.

In one embodiment of the present invention the process comprises the step of contacting an aldopentose or an aldohexose and an aldehyde or an aldehyde source with an acidic zeolite, wherein said zeolite comprises:
- at least two, preferably two or three, non-interconnected and parallel channel systems wherein, at least one of said channel systems comprises 8- or more-membered ring channels; and a framework Si/X₂ ratio of at least 4 as measured by NMR; or
- at least two, preferably two or three, interconnected and non-parallel channel systems wherein, at least one of said channel systems comprises 10- or more-membered ring channels; and a framework Si/X₂ ratio of at least 4 as measured by NMR; or
- three interconnected and non-parallel channel systems wherein at least two of the channel systems comprise 10-or more-membered ring channels, and a framework Si/X₂ ratio of at least 4 as measured by NMR,
wherein each X is Al or B.

Zeolites comprising three interconnected and non-parallel channel systems wherein at least two of the channel systems comprise 10-or more-membered ring channels, and a framework Si/X₂ ratio of at least 4 as measured by NMR lead to excellent results.

The term "channel system" preferably refers to a system of parallel and crystallographically equivalent channels, wherein the channels are 8-membered ring channels or larger.

Good results were obtained using zeolites comprising at least two interconnected and non-parallel channel systems (a 2D or 3D micropore geometry). Accordingly, the zeolites used in the process described herein comprise a 2D or 3D micropore geometry, more particularly an interconnected 2D or 3D micropore geometry.

Good results were also obtained with zeolites comprising at least one 10-or more-membered ring channel.

Accordingly, in particular embodiments, the zeolite(s) for use in the process described herein may comprise a framework Si/X2 ratio of at least 4, for example a framework Si/Al₂ ratio of at least 4, wherein the zeolite further comprises of at least two, preferably two or three, non-interconnected and parallel channel systems wherein, at least one of said channel systems comprises 8- or more-membered ring channels. An example of such zeolites, but not limited, are zeolites with mordenite (MOR) topology.

In certain embodiments, the zeolite for use in the process described herein may comprise a framework Si/X2 ratio of at least 4, for example a framework Si/ Al₂ ratio of at least 4; wherein the zeolite further comprises at least two interconnected and non-parallel channel systems wherein at least one of the interconnected and non-parallel channel systems comprise 10-or more-membered ring channels, i.e., at least one of the channel systems comprise 10-or more-membered ring channels, and at least one other channel system comprises 8-or more-membered ring channels.

In certain embodiments, the zeolite for use in the process described herein may comprise a framework Si/X2 ratio of at least 4, for example a framework Si/Al₂ ratio of at least 4; wherein the zeolite further comprises three interconnected and non-parallel channel systems wherein at least two of the interconnected and non-parallel channel systems comprise 10-or more-membered ring channels, i.e., at least two of the channel systems comprise 10-or more-membered ring channels, and the other channel system comprises 8-or more-membered ring channels. Examples of such zeolites include, but are not limited to zeolites comprising a topology selected from the group comprising BEA, FAU, and MEL.

In particular embodiments, the zeolite comprises at least two non-interconnected and parallel channel systems wherein at least one of the non-interconnected and parallel channel systems comprise 8-or more-membered ring channels; wherein the zeolite further comprises a framework Si/X2 ratio of at least4 , more particularly at least 4 , for example a ratio of at least 15, for example a ratio of at least 20, for example a ratio of at least 25, for example a ratio of at least 30, for example a ratio of at least 35, for example a ratio of at least 40, for example a ratio of at least 50, for example a ratio of at least 60, for example a ratio of at least 70, for example a ratio of at least 80, for example a ratio of at least 90, or for example a ratio of at least 100, or for example a ratio of at least 110, or for example a ratio of at least 120, or for example a ratio of at least 130, or for example a ratio of at least 140, or for example a ratio of at least 150, or for example a ratio of at least 160, or for example a ratio of at least 170, or for example a ratio of at least 180, or for example a ratio of at least 190, or for example a ratio of at least 200.

In particular embodiments, the zeolite comprises at least two, preferably two or three, interconnected and non-parallel channel systems wherein at least one of the interconnected and non-parallel channel systems comprise 10-or more-membered ring channels; wherein the zeolite further comprises a framework Si/X2 ratio of at least 4, more particularly at least 8, for example a ratio of at least 10, for example a ratio of at least 15, for example a ratio of at least 20, for example a ratio of at least 25, for example a ratio of at least 30, for example a ratio of at least 35, for example a ratio of at least 40, for example a ratio of at least 50, for example a ratio of at least 60, for example a ratio of at least 70, for example a ratio of at least 80, for example a ratio of at least 90, or for example a ratio of at least 100, or for example a ratio of at least 110, or for example a ratio of at least 120, or for example a ratio of at least 130, or for example a ratio of at least 140, or for example a ratio of at least 150, or for example a ratio of at least 160, or for example a ratio of at least 170, or for example a ratio of at least 180, or for example a ratio of at least 190, or for example a ratio of at least 200.

In particular embodiments, the zeolite comprises three interconnected and non-parallel channel systems wherein at least two of the interconnected and non-parallel channel systems comprise 10-or more-membered ring channels; wherein the zeolite further comprises a framework Si/X2 ratio of at least 4, more particularly at least 8, for example a ratio of at least 10, for example a ratio of at least 15, for example a ratio of at least 20, for example a ratio of at least 25, for example a ratio of at least 30, for example a ratio of at least 35, for example a ratio of at least 40, for example a ratio of at least 50, for example a ratio of at least 60, for example a ratio of at least 70, for example a ratio of at least 80, for example a ratio of at least 90, or for example a ratio of at least 100, or for example a ratio of at least 110, or for example a ratio of at least 120, or for example a ratio of at least 130, or for example a ratio of at least 140, or for example a ratio of at least 150, or for example a ratio of at least 160, or for example a ratio of at least 170, or for example a ratio of at least 180, or for example a ratio of at least 190, or for example a ratio of at least 200.

In most embodiments, the conversion of the sugar to be protected to the fully acetal-protected product increases as the Si/X₂ ratio increases, preferably as the Si/X₂ ratio increases. In some embodiments, it is observed that at high Si/X₂ ratios, the yield of partially protected sugars may decrease as the Si/X₂ ratio increases further. Without wishing to be bound by theory, this is believed to be related to the low amount of acid sites and yet higher Brønsted acidic strength in zeolites with high Si/X₂ ratio. Therefore, in particular embodiments, the zeolite has a framework Si/X₂ ratio below 280. In further embodiments, the zeolite has a framework Si/X₂ ratio below 150. Preferably, the zeolite has a framework Si/X₂ ratio below 280. In further embodiments, the zeolite has a framework Si/X₂ ratio below 200.

The zeolites used in the process described herein may comprise AlO₄ tetrahedra, BO₄ tetrahedra, or both. Accordingly, in some embodiments, X₂ is (Al₂ + B₂). Thus, for a given zeolite, the Si/X₂ framework ratio remains the same upon substitution of framework Al by B, or vice versa. However, it is envisaged that in particular embodiments, the zeolites may not comprise BO₄ tetrahedra, or an insignificant amount thereof (e.g., an Al/B ratio of 100 or more). Thus, in particular embodiments, X₂ may be Al₂.

The Si/X₂ ratios referred to herein are molar ratios as determined via NMR, unless specified otherwise. It will be understood by the skilled person that the Si/X₂ ratio referred to herein is equal to the SiO₂/X₂O₃ molar ratio, wherein X₂O₃ is (Al₂O₃ and/or B₂O₃). Moreover, the skilled person will understand that by dividing the Si/X₂ ratio by two, the Si/X molar ratio is obtained, wherein X is (Al and/or B).

Preferably, the channels defined by the zeolite topology are large enough to be accessible for the sugars to be protected, but small enough to prevent significant formation and/or diffusion of side products. Accordingly, in particular embodiments, the zeolite only comprises channels with a ring size of at most 18, preferably of at most 14, for example of at most 12.

In a preferred embodiment, suitable zeolites for use in the process described herein comprise a topology selected from the group comprising BEA, FAUand MEL. These zeolites result in high amounts of the final product. In certain embodiments, the zeolite(s) comprise a topology selected from the group consisting of MOR. In specific embodiments, the zeolite(s) comprise a zeolite with a FAU or BEA topology which provide excellent results.

In certain embodiments, the zeolite comprises channels having an average (equivalent) diameter of at least 5 Å. More particularly, the zeolite may comprise five or more non-parallel channels having an average diameter of at least 5 Å. The channel diameter may be determined theoretically via knowledge of the zeolite framework type, or via X-ray diffraction (XRD) measurements, as will be known by the skilled person. Preferably, the zeolite comprises two or more non-parallel and interconnected channels having an average (equivalent) diameter between 5 and 13.0 Å, more preferably between 5 and 11 Å. Preferably, the diameter for the appropriate topology is obtained from international standard literature: the Atlas of Zeolite structures or the corresponding online database, found at http://www.iza-structure.org/databases/, as referenced above. The (equivalent) diameter of the channels may also be determined experimentally via N2 adsorption, for example as discussed by Groen et al. (Microporous and Mesoporous Materials 2003, 60, 1-17), Storck et al. {Applied Catalysis A: General 1998, 174, 137-146) and Rouquerol et al. (Rouquerol F, Rouquerol J and Sing K, Adsorption by powders and porous solids: principles, methodology and applications, Academic Press, London, 1999).

In some embodiments, the zeolite may further comprise mesopores. The presence of mesopores may increase the accessibility of both sugar to be protected to the micropores, and may therefore further increase the reaction speed. However, it is also envisaged that the zeolite may not comprise mesopores.

As used herein the term "mesopores" refers to pores in the zeolite crystal having average diameters of 2.0 nm to 50 nm. For pore shapes deviating from the cylinder, the above ranges of diameter of mesopores refer to equivalent cylindrical pores. The mesopore average diameter may be determined by gas sorption techniques such as N₂ adsorption.

The zeolite(s) may be used as such, for example as a powder. In certain embodiments, the zeolite(s) may be formulated into a catalyst by combining with other materials that provide additional shape, hardness or catalytic activity to the finished catalyst product. Materials which can be blended with the zeolite may be various inert or catalytically active materials, or various binder materials. These materials include compositions such as kaolin and other clays, phosphates, alumina or alumina sol, titania, metal oxide such as zirconia, quartz, silica or silica sol, metal silicates, and mixtures thereof. These components are effective in densifying the catalyst and increasing the strength of the formulated catalyst.

The catalyst may be formulated into pellets, spheres, extruded into other shapes, or formed into spray-dried particles.

In some embodiments, the zeolite(s) for use in the processes described herein can be exposed to a (post-synthesis) treatment to increase the Si/X₂ framework ratio. Methods to increase the Si/Al₂ ratio of zeolites are known in the art, and include dealumination of the framework via (hydro)thermal treatment, extraction of framework aluminum with acid, and replacement of framework aluminum with silicon by reaction with silicon halides or hexafluorosilicates. An exemplary method of dealumination is described by Remy et al. {J. Phys. Chem. 1996, 100, 12440-12447). The zeolites for use in the process described herein preferably are Brønsted acidic zeolites, i.e., having proton donating sites in the micropores. When all Al T-sites are counterbalanced with an acidic proton (as opposed to a cation), the Brønsted acid density can be directly derived from the Si/Al₂ ratio, which is known to the skilled person.

The zeolites for use in the processes described herein can be obtained in acidic form (acidic H-form zeolite) or (partly) exchanged with a cation other than H⁺. In some embodiments, the acidic H-form zeolites can be used as such. In some other embodiments, the zeolites for use in the processes described herein can be exposed to a (post-synthesis) treatment to increase the Brønsted acid density. Brønsted acid sites in zeolites can be readily generated by aqueous ion exchange with an ammonium salt, followed by thermal decomposition of the ammonium ions inside the zeolite. Alternatively, the acid sites may be generated by aqueous ion exchange with the salt of a multivalent metal cation (such as Mg²⁺, Ca²⁺, La³⁺, or mixed rare-earth cations), followed by thermal dehydration (J. Weitkamp, Solid State Ionics 2000, 131, 175-188).

The zeolite catalysts described herein may be regenerated and reused in the process. Accordingly, particular embodiments of the process described herein may comprise a step of regenerating the zeolite catalyst. Regeneration of the zeolite catalysts can be performed via washing or calcination. Preferably, regeneration of the zeolite catalysts is done via calcination, for example at a temperature of at least 150°C. In particular embodiments, the calcination temperature is at least 200°C, for example at least 300°C, for example at least 400°C, for example at least 450°C, for example about 550°C.

The above-described zeolites may comprise various dopants such as but not limited to gallium, tin, or rare earth metals. The dopant may result in better catalyst stability, or a higher conversion of the sugar to be desired fully or partially protected products.

Exemplary commercially available zeolites suitable for use in the processes described herein include, but are not limited to, Beta polymorph A (BEA topology), Y zeolite (FAU topology), and Mordenite.

| **Catalyst designation** | **Zeolite topology** | **Zeolite ring size** | **Number of interconnected non parallel channel systems** |
|---|---|---|---|
| H-MOR | MOR | 12-8 | channel systems not interconnected (parallel channel system with 12- and 8-membered rings) |
| H-BEA | BEA | 12-12-12 | Three systems with 12-membered ring channels |
| H-Y | FAU | 12-12-12 | Three systems with 12-membered ring channels |

Preferably, the acidic zeolite catalyst is selected from the group consisting of Zeolite Y (SiO₂:Al₂O₃=5.2:1), Zeolite Y (SiO₂:Al₂O₃=12:1), Zeolite Y (SiO₂:Al₂O₃=30:1), Zeolite Y (SiO₂:Al₂O₃=60:1), Zeolite Y (SiO₂:Al₂O₃=80:1), Zeolite β (SiO₂:Al₂O₃=25:1), Zeolite β (SiO₂:Al₂O₃=38:1), Zeolite β (SiO₂:Al₂O₃=150:1) and Modernite (SiO₂:Al₂O₃=19:1).

Preferably, the aldehyde used in the process according to the present invention can be selected from the group consisting of formaldehyde, acetaldehyde, propionaldehyde, butyraldehyde, isobutyraldehyde, valeraldehyde, isovaleraldehyde, hexanal, heptanal, octanal, nonanal, decanal dodecanal, tetradecanal, hexadecanal, octadecanal, crotonaldehyde, glyoxal, malonic dialdehyde, succinic dialdehyde, glutaraldehyde, adipic dialdehyde, 2-hydroxyadipic dialdehyde, pimelic dialdehyde, suberic dialdehyde, azelaic dialdehyde, sebacic dialdehyde, maleic aldehyde, fumaric aldehyde, phthalaldehyde, isophthalaldehyde, terephthalaldehyde, and 1,4- diformylcyciohexane, glyoxylic acid, glyoxylic acid monohydrate, formyl acetic acid, terephthaldehyde, and succinaldehydic acid, preferably formaldehyde, acetaldehyde, dodecanal, glyoxylic acid, glyoxylic acid monohydrate and glutaraldehyde.

Alternatively, the aldehyde source is selected from the group consisting of paraformaldehyde, 1,3,5-trioxane, polyoxymethylene, and metaldehyde. Said polymeric or oligomeric compounds form under reaction conditions the corresponding aldehydes.

Thus, the aldehydes can be a gas, a liquid or a solid, and may be present as solution or as pure compounds. For example, in the case of formaldehyde, it can be provided for example as gas, as formaldehyde solutions (e.g., formalin), as paraformaldehyde, or as 1,3,5-trioxane, polyoxymethylene (POM).

In one embodiment of the present invention R₁ and R₁', R₂ and R₂', R₃ and R₃', and R₁₂ and R₁₂' are identical. Said compounds can be obtained with the same aldehyde results in a simple synthesis protocol.

In another embodiment of the present invention R₁ and R₁', R₂ and R₂', R₃ and R₃', and R₁₂ and R₁₂' are different from each other. For example, in a first step the sugar to be protected reacts with a first aldehyde (i.e. dodecanal) in the presence of a heterogenous acidic catalyst to protect one side of the sugar. Afterwards, in a second step this partially protected sugar reacts in the presence of heterogenous acidic catalyst with a different aldehyde (for example glyoxylic acid) to obtain a fully protected sugar.

In one embodiment according to the present invention sugar to be protected is an aldopenose resulting in a compound of formula I, IV, V, IX, X and XI 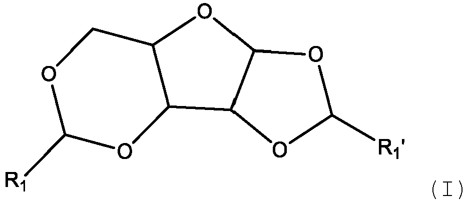 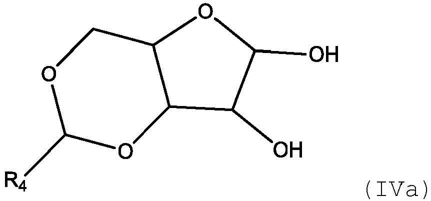 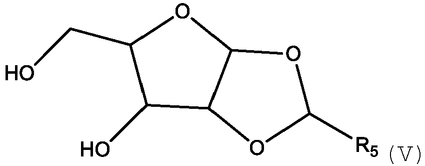 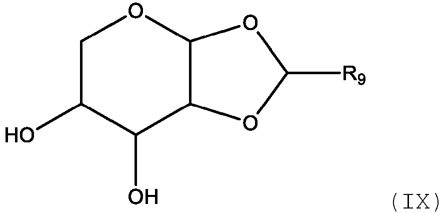 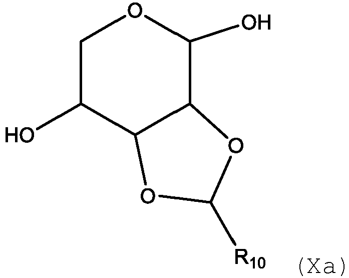 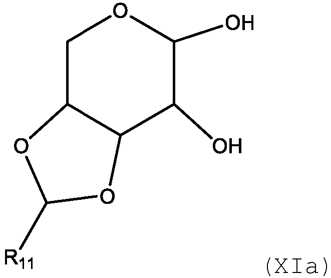 wherein R₁, R₁', R₄, R₅, R₉, R₁₀ and R₁₁ have the same definitions as above.

Exemplary reactions to obtain the compounds of formula I, IV, V, IX, X or XI in the presence of a heterogenous acidic catalyst are:

| Starting compound | Aldehyde | Examplary reaction | Exemplary product |
|---|---|---|---|
| 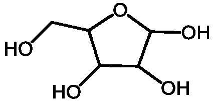 | Linear aldehyde | 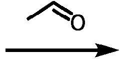 | 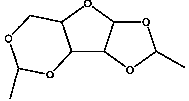 |
| 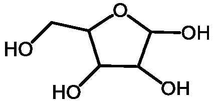 | Branched aldehyde | 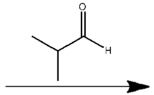 | 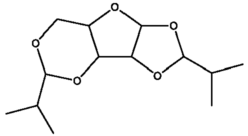 |
| 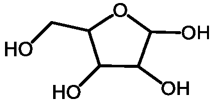 | Linear aldehyde with double bond | 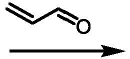 | 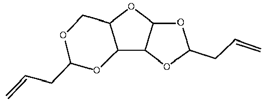 |
| 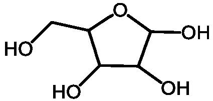 | Alkoxyalkylaldehyde | 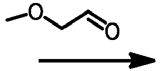 | 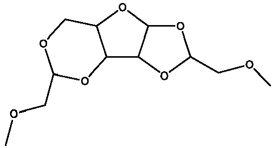 |
| 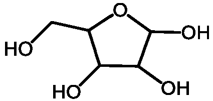 | Aldoester | 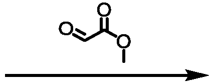 | 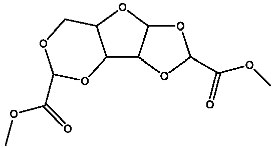 |
| 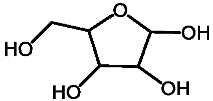 | Aldoacid | 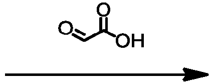 | 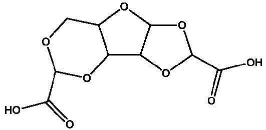 |
| 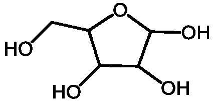 | Two different aldehydes | 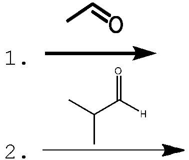 | 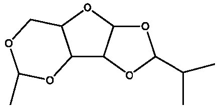 |
| 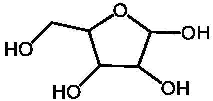 | Dialdehyd e | 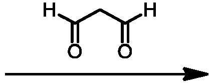 | 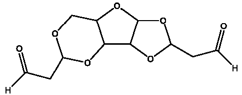 |
| 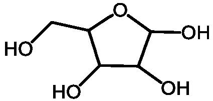 | Linear aldehyde | 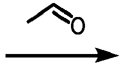 | 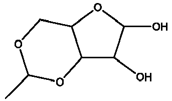 |
| 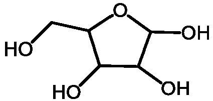 | Branched aldehyde | 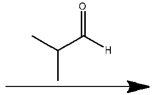 | 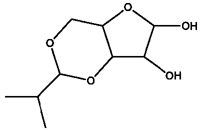 |
| 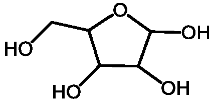 | Linear aldehyde with double bond | 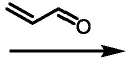 | 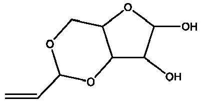 |
| 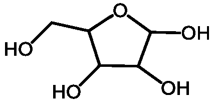 | Alkoxyalkylaldehyde | 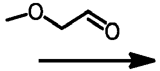 | 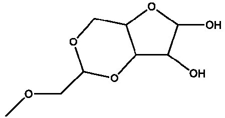 |
| 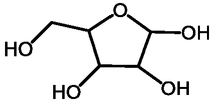 | Aldoester | 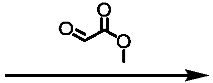 | 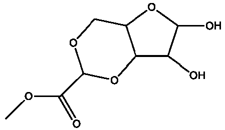 |
| 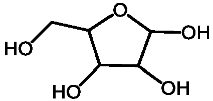 | Aldoacid | 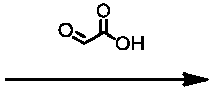 | 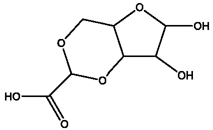 |
| 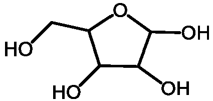 | Dialdehyd e | 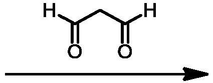 | 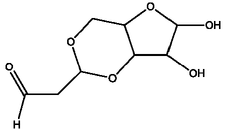 |
| 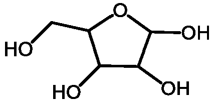 | Linear aldehyde | 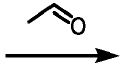 | 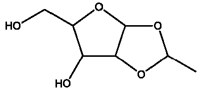 |
| 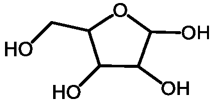 | Branched aldehyde | 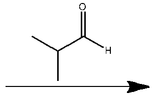 | 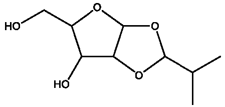 |
| 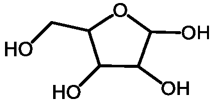 | Linear aldehyde with double bond | 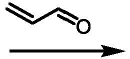 | 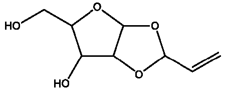 |
| 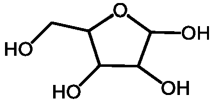 | Alkoxy-alkylaldehyde | 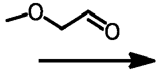 | 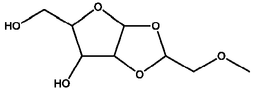 |
| 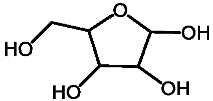 | Aldoester | 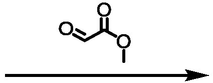 | 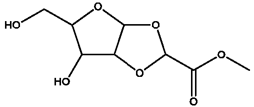 |
| 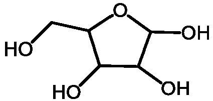 | Aldoacid | 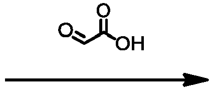 | 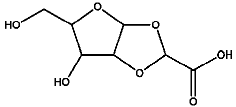 |
| 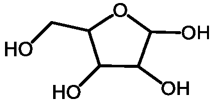 | Dialdehyde | 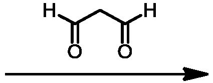 | 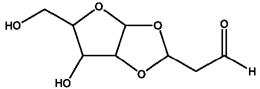 |
| 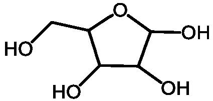 | Linear aldehyde | 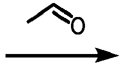 | 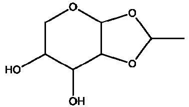 |
| 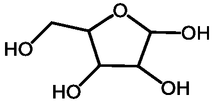 | Branched aldehyde | 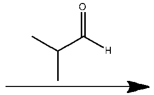 | 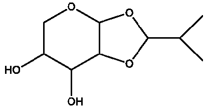 |
| 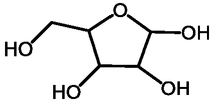 | Linear aldehyde with double bond | 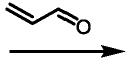 | 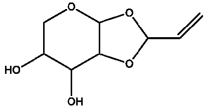 |
| 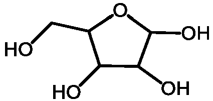 | Alkoxyalkylaldehyde | 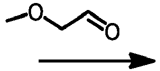 | 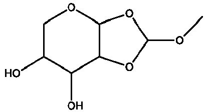 |
| 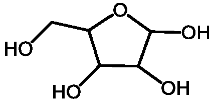 | Aldoester | 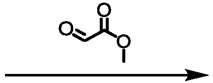 | 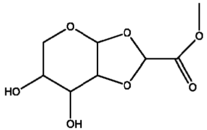 |
| 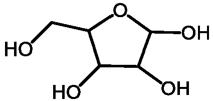 | Aldoacid | 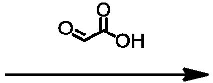 | 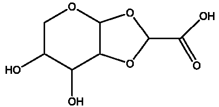 |
| 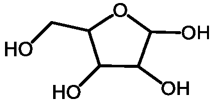 | Dialdehyd e | 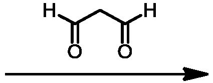 | 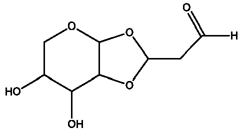 |
| 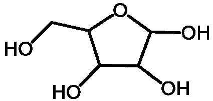 | Linear aldehyde | 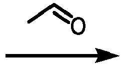 | 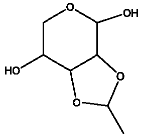 |
| 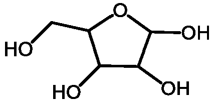 | Branched aldehyde | 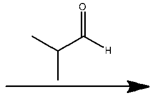 | 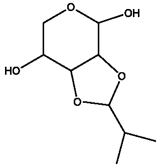 |
| 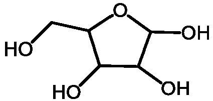 | Linear aldehyde with double bond | 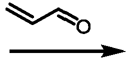 | 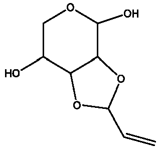 |
| 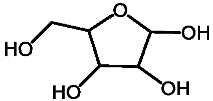 | Alkoxyalkylaldehyde | 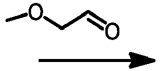 | 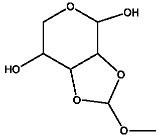 |
| 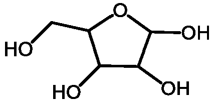 | Aldoester | 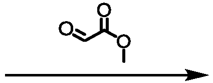 | 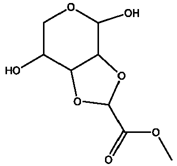 |
| 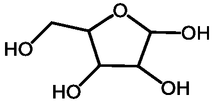 | Aldoacid | 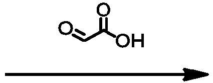 | 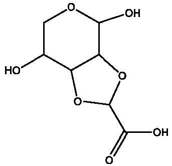 |
| 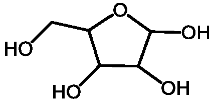 | Dialdehyd e | 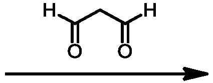 | 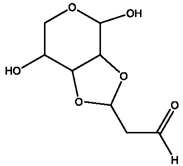 |
| 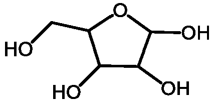 | Linear aldehyde | 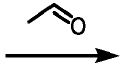 | 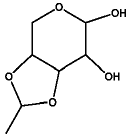 |
| 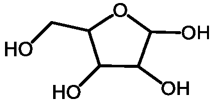 | Branched aldehyde | 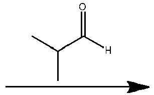 | 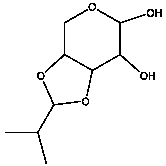 |
| 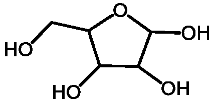 | Linear aldehyde with double bond | 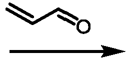 | 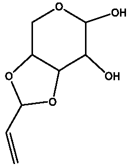 |
| 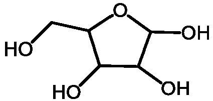 | Alkoxyalkylaldehyde | 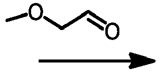 | 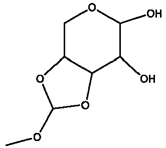 |
| 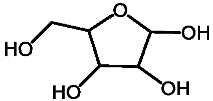 | Aldoester | 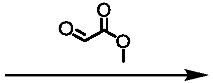 | 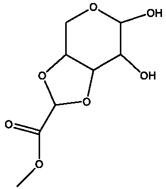 |
| 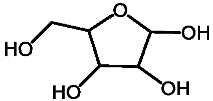 | Aldoacid | 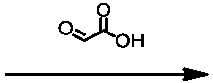 | 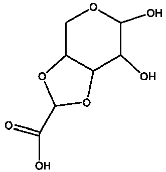 |
| 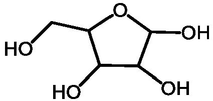 | Dialdehyd e | 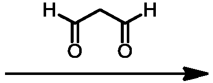 | 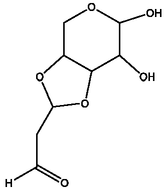 |

The aldopentose can be selected from the group consisting of D-ribose, L-ribose, D-arabinose, L-arabinose, D-xylose, L-Xylose, D-Lyxose and L-Lysose, most preferably from D-arabinose and D-xylose and ideally D-xylose. The stereochemistry of aldopentoses is known to the skilled person.

In one embodiment according to the present invention sugar to be protected is an aldopentoside resulting in a partially protected sugar selected from a compound of IVb, Xb or XIb 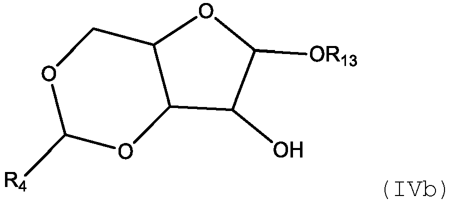 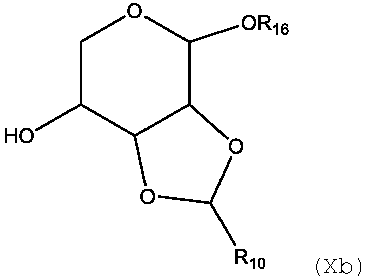 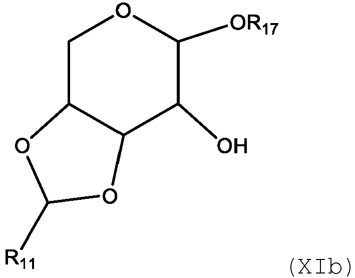
wherein R₄, R₁₀ and R₁₁ have the same definitions as above and
R₁₃, R₁₆ and R₁₇ are independent from each other hydrogen or a linear, branched or cyclic hydrocarbon moiety having 1 to 20 carbon atoms, preferably methyl, ethyl, propyl, or butyl.

Exemplary reactions to obtain the compounds of formula IVb, Xb or XIb in the presence of a heterogenous acidic catalyst are:

| Starting compound | Aldehyde | Examplary reaction | Exemplary product |
|---|---|---|---|
| 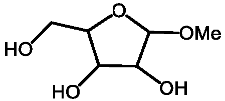 | Linear aldehyde | 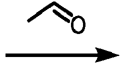 | 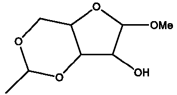 |
| 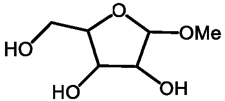 | Branched aldehyde | 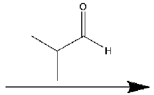 | 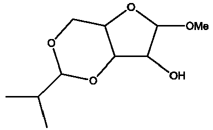 |
| 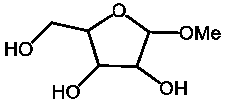 | Linear aldehyde with double bond | 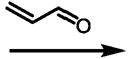 | 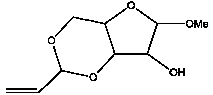 |
| 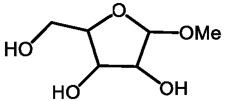 | Alkoxyalkylaldehyde | 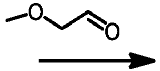 | 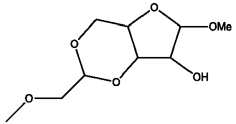 |
| 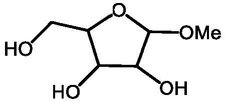 | Aldoester | 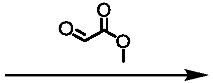 | 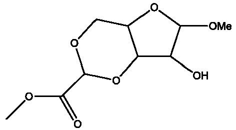 |
| 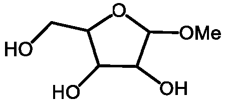 | Aldoacid | 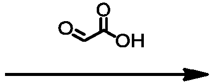 | 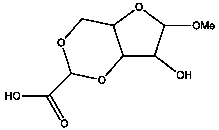 |
| 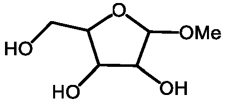 | Dialdehyde | 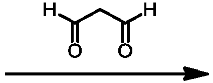 | 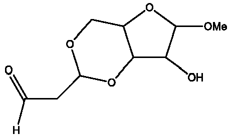 |
| 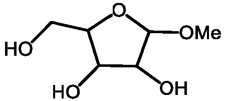 | Linear aldehyde | 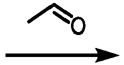 | 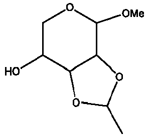 |
| 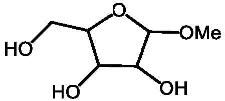 | Branched aldehyde | 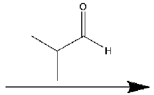 | 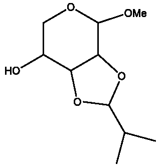 |
| 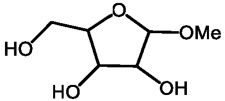 | Linear aldehyde with double bond | 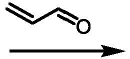 | 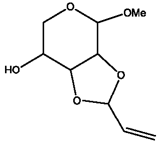 |
| 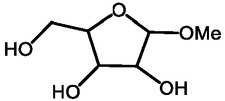 | Alkoxyalkylaldehyde | 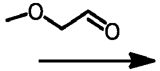 | 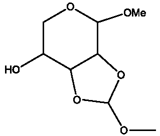 |
| 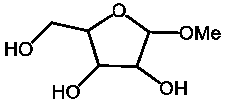 | Aldoester | 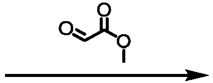 | 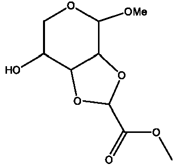 |
| 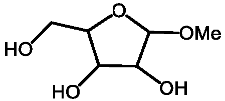 | Aldoacid | 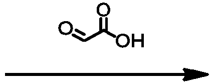 | 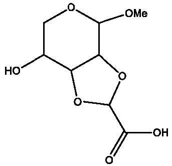 |
| 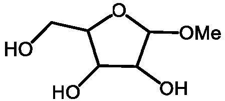 | Dialdehyde | 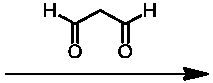 | 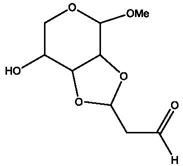 |
| 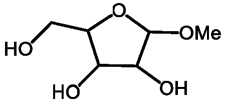 | Linear aldehyde | 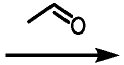 | 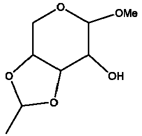 |
| 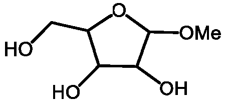 | Branched aldehyde | 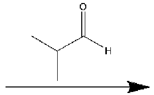 | 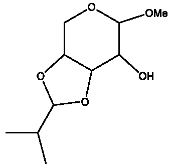 |
| 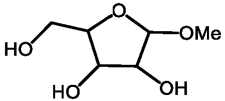 | Linear aldehyde with double bond | 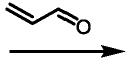 | 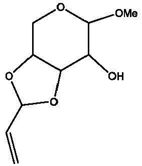 |
| 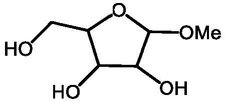 | Alkoxyalkylaldehyde | 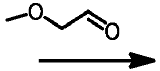 | 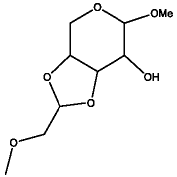 |
| 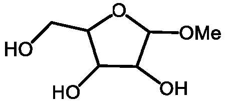 | Aldoester | 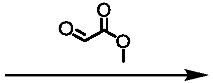 | 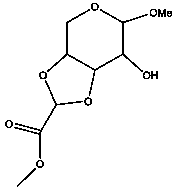 |
| 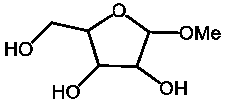 | Aldoacid | 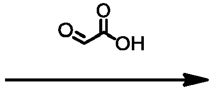 | 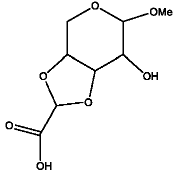 |
| 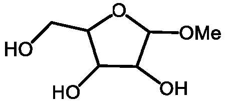 | Dialdehyde | 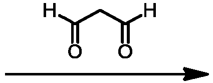 | 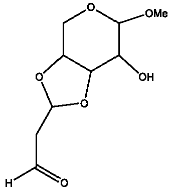 |

In another embodiment according to the present invention sugar to be protected is an aldohexose resulting in a compound of formula II, III VIa, VII, VIIIa 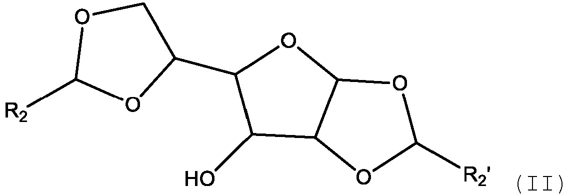 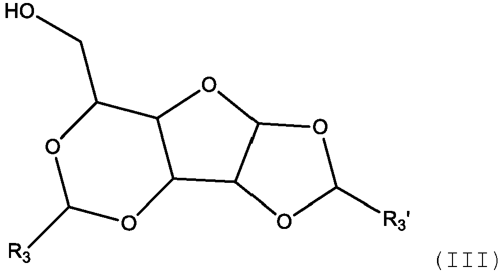 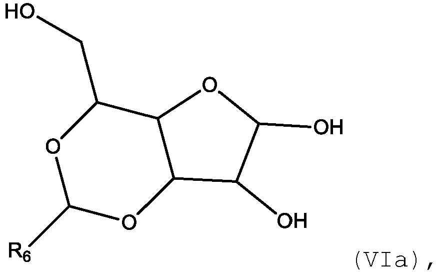 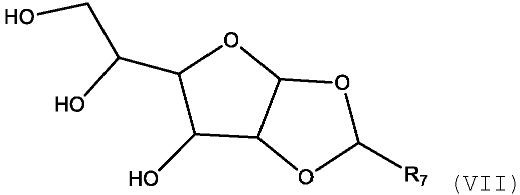 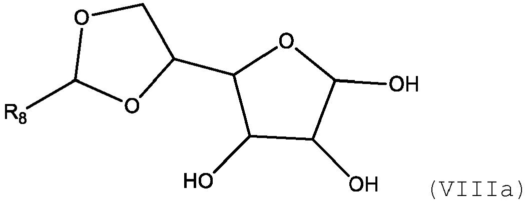 wherein R₂, R₂', R₃, R₃', R₆, R₇, and R₈ have the same definitions as above.

The aldohexose is preferably selected from the group consisting of D-allose, L-allose, D-altrose, L-altrose, D-glucose, L-glucose, D-mannose, L-mannose, D-gulose, L-gulose, D-idose, L-idose, D-galactose, L-galactose, D-talose, and L-talose, most preferably from D-glucose. The stereochemistry of aldohexose is known to the skilled person.

Exemplary reactions to obtain the compounds of formula II, III, VIa, VII and VIIIa in the presence of a heterogenous acidic catalyst are:

| | | | |
|---|---|---|---|
| 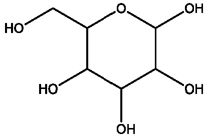 | Linear aldehyde | 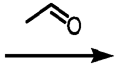 | 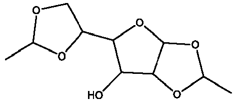 |
| 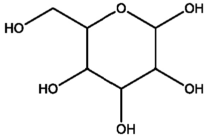 | Branched aldehyde | 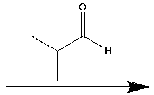 | 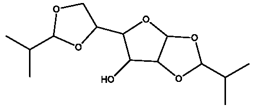 |
| 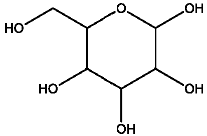 | Linear aldehyde with double bond | 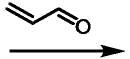 | 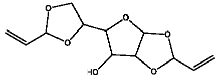 |
| 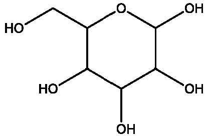 | Alkoxyalkylaldehyde | 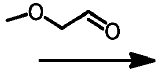 | 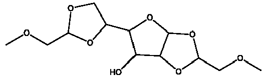 |
| 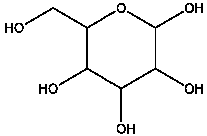 | Aldoester | 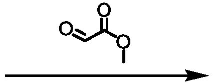 | 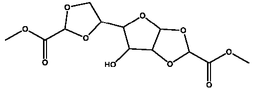 |
| 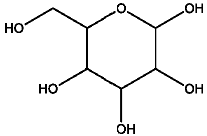 | Aldoacid | 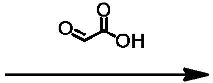 | 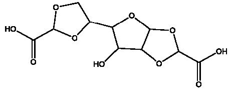 |
| 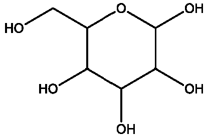 | Dialdehyde | 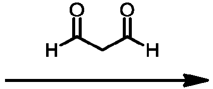 | 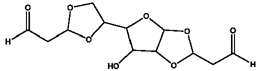 |
| 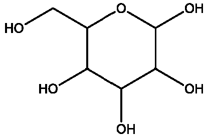 | Linear aldehyde | 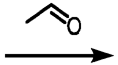 | 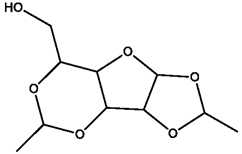 |
| 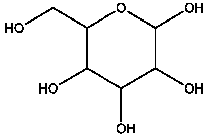 | Branched aldehyde | 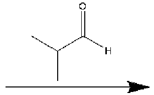 | 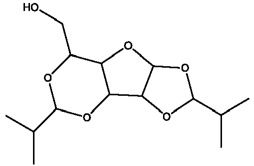 |
| 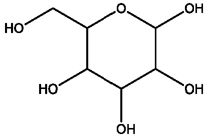 | Linear aldehyde with double bond | 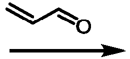 | 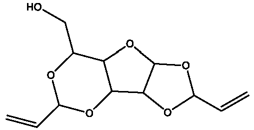 |
| 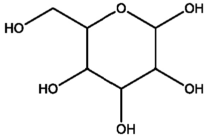 | Alkoxyalkylaldehyde | 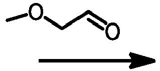 | 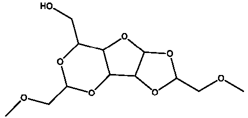 |
| 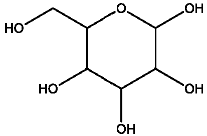 | Aldoester | 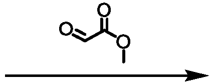 | 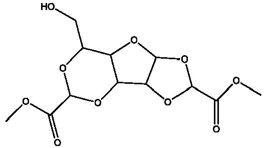 |
| 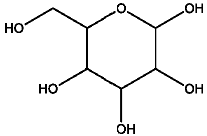 | Aldoacid | 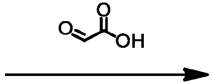 | 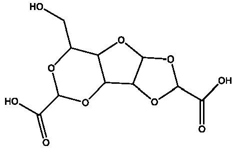 |
| 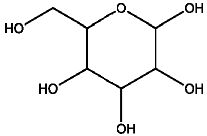 | Dialdehyde | 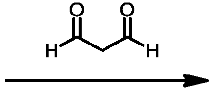 | 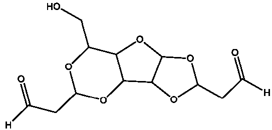 |
| 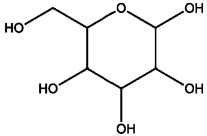 | Linear aldehyde | 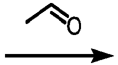 | 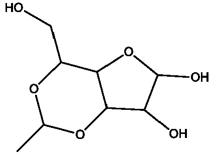 |
| 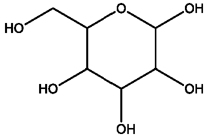 | Branched aldehyde | 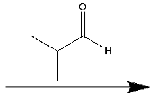 | 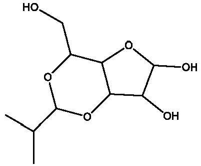 |
| 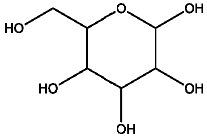 | Linear aldehyde with double bond | 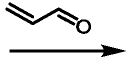 | 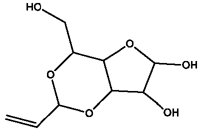 |
| 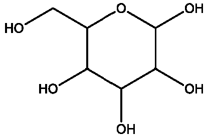 | Alkoxyalkylaldehyde | 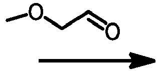 | 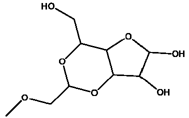 |
| 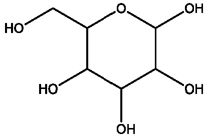 | Aldoacid | 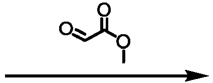 | 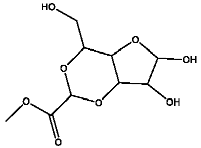 |
| 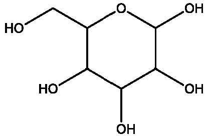 | Aldoacid | 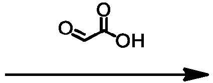 | 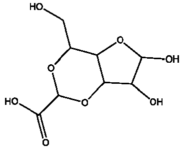 |
| 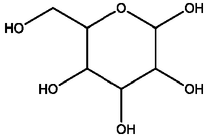 | Dialdehyde | 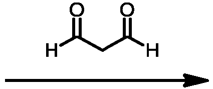 | 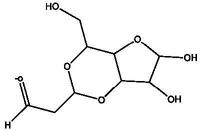 |
| 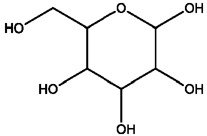 | Linear aldehyde | 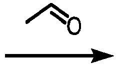 | 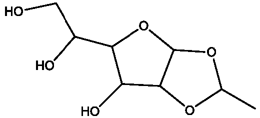 |
| 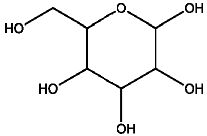 | Branched aldehyde | 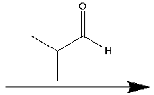 | 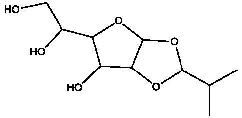 |
| 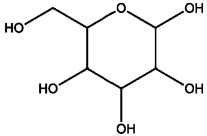 | Linear aldehyde with double bond | 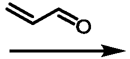 | 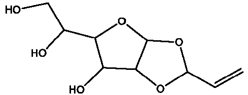 |
| 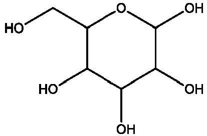 | Alkoxyalkylaldehyde | 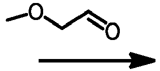 | 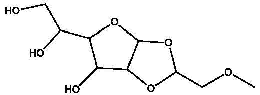 |
| 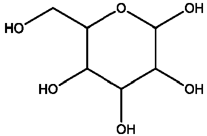 | Aldoester | 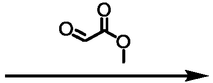 | 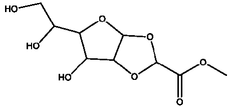 |
| 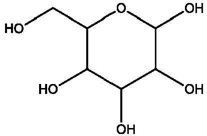 | Aldoacid | 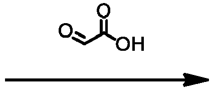 | 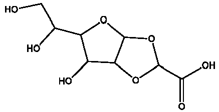 |
| 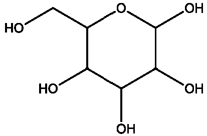 | Dialdehyde | 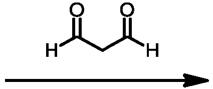 | 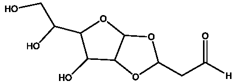 |
| 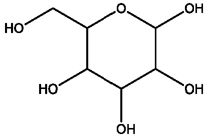 | Linear aldehyde | 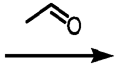 | 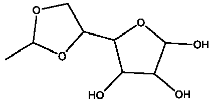 |
| 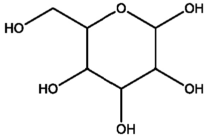 | Branched aldehyde | 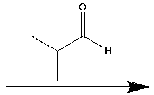 | 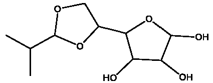 |
| 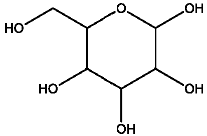 | Linear aldehyde with double bond | 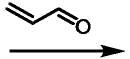 | 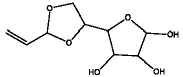 |
| 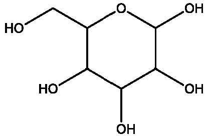 | Alkoxyalkylaldehyde | 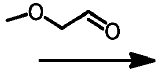 | 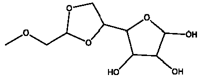 |
| 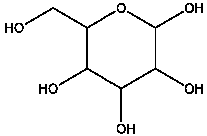 | Aldoester | 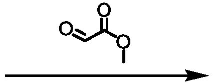 | 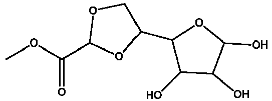 |
| 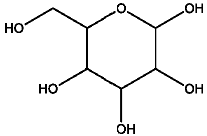 | Aldoacid | 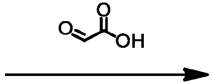 | 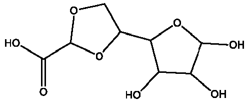 |
| 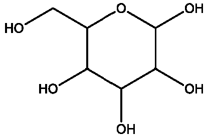 | Dialdehyde | 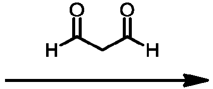 | 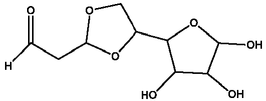 |

In one embodiment according to the present invention sugar to be protected is an aldohexoside resulting in an at least partially protected sugar selected from a compound of VIb, VIIIb, and XII: 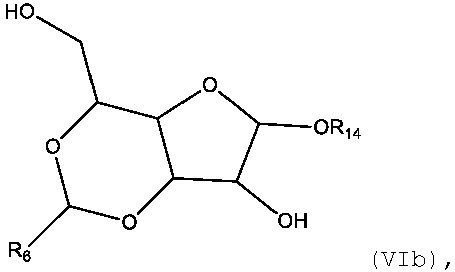 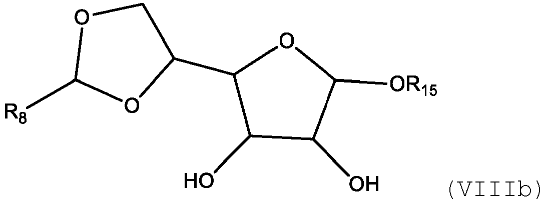 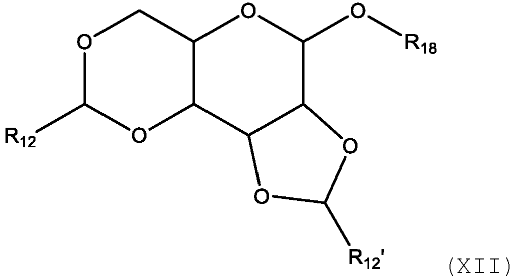
wherein R₆, R₈, R₁₂ and R₁₂' have the same definitions as above and
R₁₄, R₁₅ and R₁₈ are independent from each other hydrogen or a linear, branched or cyclic hydrocarbon moiety having 1 to 20 carbon atoms, preferably methyl, ethyl or propyl.

Exemplary reactions to obtain the compounds of formula VIb, VIIIb and VII in the presence of a heterogenous acidic catalyst are:

| | | | |
|---|---|---|---|
| 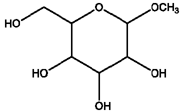 | Linear aldehyde | 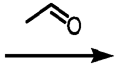 | 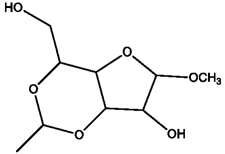 |
| 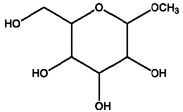 | Branched aldehyde | 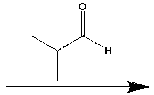 | 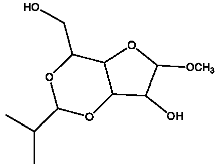 |
| 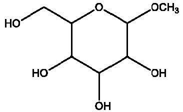 | Linear aldehyde with double bond | 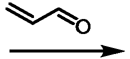 | 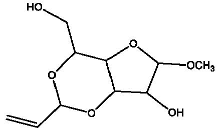 |
| 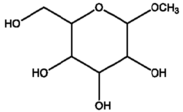 | Alkoxyalkylaldehyde | 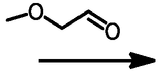 | 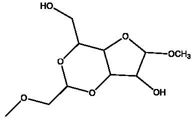 |
| 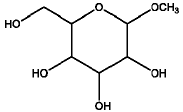 | Aldoacid | 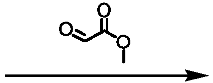 | 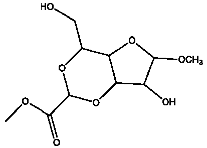 |
| 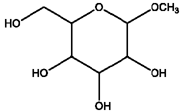 | Aldoacid | 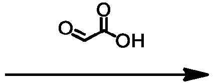 | 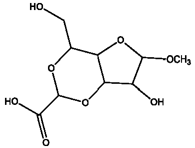 |
| 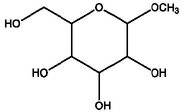 | Dialdehyd e | 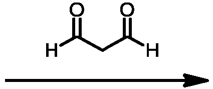 | 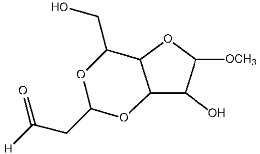 |
| 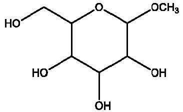 | Linear aldehyde | 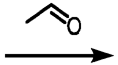 | 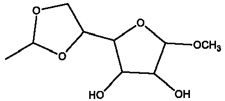 |
| 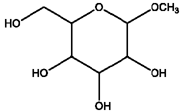 | Branched aldehyde | 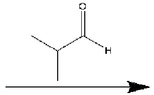 | 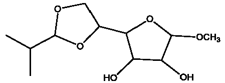 |
| 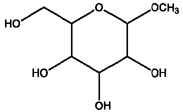 | Linear aldehyde with double bond | 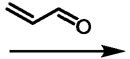 | 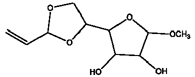 |
| 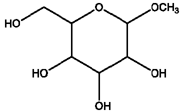 | Alkoxyalkylaldehyde | 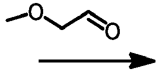 | 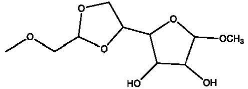 |
| 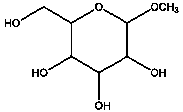 | Aldoester | 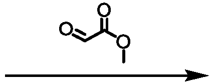 | 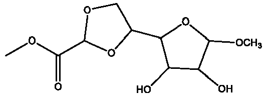 |
| 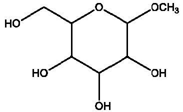 | Aldoacid | 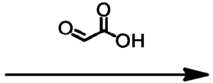 | 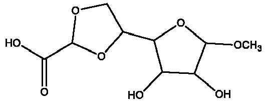 |
| 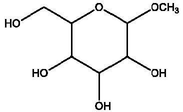 | Dialdehyd e | 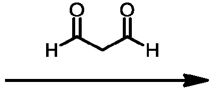 | 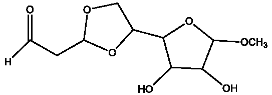 |
| 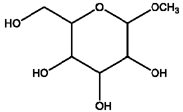 | Linear aldehyde | 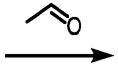 | 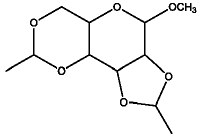 |
| 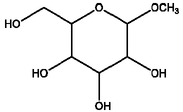 | Branched aldehyde | 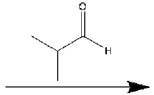 | 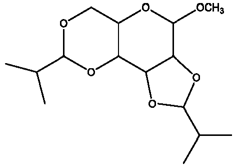 |
| 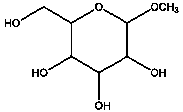 | Linear aldehyde with double bond | 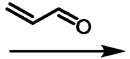 | 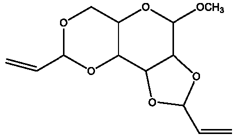 |
| 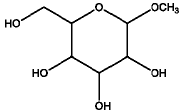 | Alkoxyalkylaldehyde | 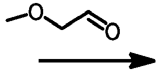 | 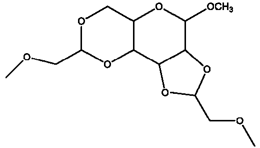 |
| 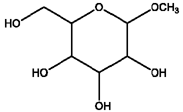 | Aldoester | 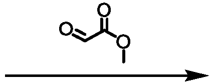 | 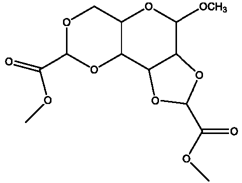 |
| 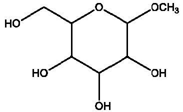 | Aldoacid | 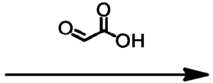 | 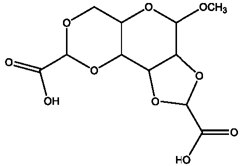 |
| 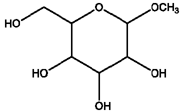 | Aldoacid | 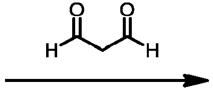 | 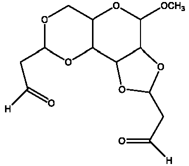 |

Preferably, the catalyst has a pore structure such as described for zeolites above in detail. The pore shape selectivity of heterogeneous catalysts allows the product selectivity to be tuned and avoid the common issue in homogeneous catalyst systems that sugar molecules with their many OH groups and the ring system tend to form many side products and therefore it is difficult to obtain a high selectivity towards one desired product.

For compounds of the formula I, II, III or XII there is no limitation with regard to the pore size, as along as the pore size permits reactants to access the Brønsted acid sites and the fully acetal-protected sugars to diffuse out. The pore size should also be large enough to allow products to leave the catalyst after reaction. If high selectivity towards partially protected sugars is preferred, a pore size should be greater than the size of the desired partially protected sugar yet smaller than the fully protected alternative. The exact pore size depends on the type of sugar and aldehyde used in the reaction. The skilled person can predict the range of pore sizes being used in these cases. Due to steric reasons only unprotected or partially protected aldopentoses, aldopentoside, aldohexoses, and aldohexoside can pass through the pores to the catalytically active center and leave the pores afterwards, and thus such catalysts allow to prepare compounds of the formula IV (IVa and IVb), V, VI (VIa and VIb), VII, VIII (VIIIa and VIIIb), IX, X (Xa and Xb)and XI (XIa and XIb).However, to obtain higher yields, pore sizes of 0.7 nm or more are preferred for aldohexose and aldohexoside protection and of 0.5 or more for aldopentose and aldopentoside protection, and catalysts comprising mesopores having average diameters of 2 nm to 50 nm.

In one embodiment according to the present invention the reaction is carried out in an organic solvent. Thus, the sugar selected from the group consisting of an aldopentose, an aldohexose, an aldopentoside and an aldohexoside and the aldehyde or the aldehyde source can react in either a batch reactor or a continuous reactor, preferably in a flow reactor, in the presence of the above-mentioned heterogenous Brønsted acidic catalysts and said organic solvent. The heterogenous Brønsted acidic catalyst used is preferably essentially insoluble in the organic solvent. After carrying out the reaction the catalyst can be removed for example by filtration and recycled and fed back to the reactor if the catalyst is not already restrained inside the reactor, such as in a basket or column packing. The remaining reaction mixture can be concentrated by evaporating the solvent. The final product can be obtained by crystallization from the concentrated liquor. Preferably the organic solvent is selected from the group consisting of consisting of dimethylisosorbide, cyclic ethers, in particular 1,4-dioxane, 2-methyltetrahydrofuran, sulfolane, sulfolene, aliphatic acids, in particular acetic acid, alkylpyrrolidones, cyclic carbonates, cyclic esters, in particular γ-valerolactone, γ-butyrolactone, acetonitrile, dialkylethers, in particular CPME and diethylether, cyclic ethers, and glycol monoethers and glycoldiethers.

Said solvents can be removed, while at the same time allowing a high conversion rate of the sugar to be protected. This solvent system is especially preferred in case of aldehydes with a long chain (for example decanal).

In another embodiment the reaction is carried out in an aqueous solution. Thus, the sugar selected from the group consisting of an aldopentose, an aldohexose, an aldopentoside and an aldohexoside and the aldehyde or the aldehyde source can react in either a batch reactor or a continuous reactor, preferably in a flow reactor, in the presence of the above-mentioned heterogenous Brønsted acidic catalysts and water. The heterogenous Brønsted acidic catalyst used is essentially insoluble in water. After the reaction, the catalyst can be separated and recycled by filtration and/or centrifugal separation if the catalyst is not already restrained inside the reactor, such as in a basket or column packing. The remaining liquid can be extracted with solvents (e.g. hexane, DCM, diethyl ether, ethyl acetate or CPME) to remove the product. The extractive solvent can be then removed by evaporation to separate the product. Alternatively, the product containing extractive layer can be concentrated to allow the product to gradually crystalize in the concentrated liquor. The extracted aqueous phase contains unreacted reactants, i.e. the sugar, the aldehyde and intermediates. Said extracted aqueous phase can be concentrated and recycled to the reactor to improve overall yield. For environmental reasons, an aqueous solution is particularly preferred as a solvent system.

In one embodiment of the present invention the reaction is carried out in a biphasic solvent system in order to achieve *in situ* separation of the product from the reaction mixture and improve the product yield by shifting the reaction equilibrium. In details, an extractant phase is added together with the reaction mixture and the heterogeneous acidic catalyst to a batch reactor. In continuous flow reactors, the extractant can be added in either a co-current, cross-current, or counter-current manner. Preferably said biphasic solvent system is selected from the group consisting of dialkyl ether/water (e.g., CPME/water, dialkylethers/water), anisole/water, dialkyl ketone/water (such as methylisobutylketone), and toluene/water, preferably CPME/water, toluene/water, and most preferably CPME/water. The reaction takes place in the aqueous phase and the product is extracted to the organic layer during the reaction. For stability reasons biphasic solvent systems selected from the group consisting of dialkyl ether/waterand dialkyl ketone/water are more suitable for aldehydes with no or a short side chain such as formaldehyde and acetaldehyde. The product containing extractant phase is concentrated to crystalize the product. The remaining product in the aqueous phase can be further extracted with solvents (e.g., ethyl acetate or CPME) to remove product which can also be crystalized after evaporating the extractant. The extracted aqueous phase can be concentrated and recycled as described above.

Preferably, the reaction is carried out at a temperature of 50 to 160°C, most preferably 80 to 140°C. In an organic solvent, the reaction temperature is typically between 80 and 130°C, in a biphasic solvent system between 120°C and 140°C and in an aqueous solution preferably between 120°C and 150°C. The reaction time is determined by the degree of conversion reached.

The amount of the heterogeneous acidic catalyst used is based on the amount of the sugar to be protected. For the alternative, and preferred, continuous operational mode, the relative amount of catalyst will be adjusted to the size of the reactor and the flow of the aldopentose or the aldohexose. In this case it will be appreciated that the determination of the appropriate relative amount based on the figures for the batchwise operational mode is within the normal skill of the production chemist or a chemical engineer.

The method according to the present invention is conveniently carried out in a normal environment, but it can also be carried out under an inert gas atmosphere, preferably under gaseous nitrogen, helium, or argon.

### Examples

The following examples 1 to 5 are based on the use of formaldehyde to produce DFX. The same principle can be applied with other aldehyde to produce different types of fully or partially protected xylose in particular for dipropyl xylose, di-n-butyl xylose, diisobutyl xylose, didodecyl xylose, etc.

### Example 1:

D-xylose (2g, 13.3 mmol, 1.0 equiv.), paraformaldehyde (2 g, 66.7 mmol formaldehyde, 5.0 equiv.) and H-form Y type zeolite (SiO₂:Al₂O₃=80:1, 2 g) were added to 2-Me-THF (32 mL) in a 50 mL round bottom flask. The mixture was then heated to 120 °C for 6 h with stirring. The resulting solution was cooled to room temperature (~23-25°C), filtered with a nylon membrane filter, and concentrated in vacuo using a rotary evaporator with a bath temperature of 45 °C. HPLC measurement showed 82.3% DFX yield from D-xylose. The concentrated residue crystallized at 4-5°C.

Other catalysts can also be used using this same method. Table 1 shows the different DFX yield using different catalysts. These reactions were conducted with 0.25g of D-xylose and all other chemicals proportionally scaled down from the above example. The reactions were conducted in 10mL glass reactors.

**Table 1. DFX yield and xylose conversion using various heterogenous catalysts and reaction time with paraformaldehyde as the source of formaldehyde**

| Catalyst | Temperature (°C) | Reaction time (h) | DFX yield (%) | Xylose conversion (%) |
|---|---|---|---|---|
| H-form Zeolite Y (SiO₂:Al₂O₃=5.2:1) | 110 | 6 | 55 | 99 |
| H-form Zeolite Y (SiO₂:Al₂O₃=12:1) | 110 | 6 | 52 | 98 |
| H-form Zeolite Y (SiO₂:Al₂O₃=30:1) | 110 | 6 | 65 | 98 |
| H-form Zeolite Y (SiO₂:Al₂O₃=60:1) | 110 | 6 | 77 | 98 |
| H-form Zeolite Y (SiO₂:Al₂O₃=80:1) | 120 | 6 | 85 | 99 |
| H-form Zeolite β (SiO₂:Al₂O₃=25:1) | 120 | 24 | 33 | 97 |
| H-form Zeolite β (SiO₂:Al₂O₃=38:1) | 120 | 24 | 50 | 98 |
| H-form Zeolite β (SiO₂:Al₂O₃=150:1) | 120 | 24 | 60 | 97 |
| H-form Modernite (SiO₂:Al₂O₃=19:1) | 120 | 24 | 19 | 80 |

### Example 2:

D-xylose (0.1g, 0.67 mmol, 1.0 equiv), paraformaldehyde (0.1 g, 3.34 mmol, 5 equiv) and ZrO₂/SO₄²⁻ (self-synthesized, 50 mg) were added to 2-Me-THF (2 mL) in a 10 mL glass reactor. The mixture was then heated to 110 °C for 9 h with stirring. The resulting solution was cooled to room temperature (~23-25°C), filtered with a nylon membrane filter and diluted 10 times in distilled water. HPLC measurement showed 79% DFX yield from D-xylose.

Other acidic catalyst groups (e.g. Amberlite, Sulphated ZrO₂, Heteropolyacids, Niobia oxides) can also be used using this same method (Table 2).

**Table 2. DFX yield and xylose conversion using various heterogenous catalysts and reaction time with paraformaldehyde as the source of formaldehyde**

| Catalyst | Temperature (°C) | Reaction time (h) | DFX yield (%) | Xylose conversion (%) |
|---|---|---|---|---|
| Sulfated zirconia | 110 | 9 | 79 | 100 |
| Orthorhombic niobium oxide | 110 | 24 | 6 | 100 |
| Phosphotungs tic acid | 110 | 1.5 | 81 | 100 |
| Amberlite IRC120 | 110 | 4 | 83 | 100 |

### Example 3:

D-xylose (0.1g, 0.67 mmol, 1.0 equiv.), formalin 37% aq. (0.5 ml, 10.3 equiv.) and β-type zeolite (SiO₂:Al₂O₃=25:1, 0.1 g) were added to GVL (5 mL) in a 10 mL glass reactor. The mixture was then heated to 140 °C for 2 h with stirring. The resulting solution was cooled to room temperature (~23-25°C), filtered with a nylon membrane filter, and distilled at 80°C under reduced pressure (10mbar) to remove GVL. HPLC measurement showed 76% DFX yield from D-xylose. The concentrated residue crystallized at 4-5°C.

Other catalysts and solvents can also be used using this same method. Table 3 shows the different DFX yield using other catalysts.

**Table 3. DFX yield and xylose conversion using various heterogenous catalysts and reaction time with formalin (formaldehyde aqueous solution) as the source of formaldehyde**

| Catalyst | Solvent | Temperature (°C) | Reaction time (h) | DFX yield (%) | Xylose conversion (%) |
|---|---|---|---|---|---|
| Amberlyst-15 | GVL | 110 | 24 | 81 | 100 |
| Aluminum chloride | GVL | 110 | 24 | 60.4 | 100 |
| H-form Modernite (SiO₂:Al₂O₃=19:1) | 1,4-dioxane | 110 | 24 | 41 | 73 |
| H-form Zeolite β (SiO₂:Al₂O₃=25:1) | Acetonitrile | 130 | 24 | 59 | 97 |
| H-form Zeolite β (SiO₂:Al₂O₃=25:1) | GVL | 140 | 2 | 76 | 100 |
| H-form Zeolite β (SiO₂:Al₂O₃=25:1) | 1,4-dioxane | 130 | 24 | 63 | 99 |
| H-form Zeolite β (SiO₂:Al₂O₃=25:1) | 1,4-dioxane | 110 | 48 | 82 | 99 |
| H-form Zeolite Y (SiO₂:Al₂O₃=30:1) | 1,4-dioxane | 120 | 24 | 59 | 99 |

### Example 4:

D-xylose (8g, 53.3 mmol, 1.0 equiv.), formalin 37% aq. (40 ml, 10.3 equiv.) and Y type zeolite (SiO₂:Al₂O₃=80:1, 1.6 g) were mixed in a 60 mL glass reactor. The mixture was then heated to 140 °C for 6 h with stirring. HPLC measurement shows 57.6% DFX yield from D-xylose. The resulting solution was cooled to room temperature (~23-25°C), filtered with a nylon membrane filter. The filtrate was extracted four times with 10 ml of ethyl acetate or cyclopentyl methyl ether in a separatory funnel. The resulting organic layer was concentrated at 45 °C under reduced pressure (0.02 mbar) to obtain a DFX-rich liquor. The DFX in the liquor was then crystallized at 4-5°C or room temperature.

Other catalysts can also be used using this same method. Table 4 shows the different DFX yield using other catalysts.

**Table 4. DFX yield and xylose conversion using various heterogenous catalysts and reaction time using the example 4**

| Catalyst | Temperature (°C) | Reaction time (h) | DFX yield (%) | Xylose conversion (5) |
|---|---|---|---|---|
| H-form Zeolite β (SiO₂:Al₂O₃=25:1) | 110 | 2 | 43 | 97 |
| H-form Zeolite β (SiO₂:Al₂O₃=38:1) | 110 | 2 | 50 | 97 |
| H-form Modernite (SiO₂:Al₂O₃=19: 1) | 140 | 2 | 29 | 79 |
| H-form Zeolite Y (SiO₂:Al₂O₃=80:1) | 140 | 2 | 50 | 90 |
| H-form Zeolite Y (SiO₂:Al₂O₃=80:1) | 120 | 24 | 59 | 95 |
| H-form Zeolite Y (SiO₂:Al₂O₃=80:1) | 100 | 24 | 37 | 76 |
| Amberlyst 36 | 140 | 2 | 64 | 96 |

### Example 5:

D-xylose (0.3g, 2 mmol, 1.0 equiv.), formalin 37wt% aq. (1.5 ml, 10.3 equiv.) and Y type zeolite (SiO2:Al2O3=80:1, 0.2 g) were mixed in a 10 mL glass reactor. Cyclopentyl methyl ether (4.5 ml, 3 vol. equiv.). was added to the aqueous layer. The mixture was then heated to 140 °C for 4 h with stirring. HPLC measurement shows 50.4% DFX yield in the organic cyclopentyl methyl ether layer and 18.4% DFX yield in the aqueous layer. The solution was cooled to room temperature (~23-25°C). The aqueous and organic layers were separated and filtered with nylon membrane filters. The organic layer was distilled at 45 °C, under reduced pressure (0.02 mbar) to obtain a concentrated liquor. The aqueous filtrate was extracted three times with 1.5 ml of ethyl acetate or cyclopentyl methyl ether in a separatory funnel. The extractant layer was distilled at 45 °C, under reduced pressure (0.02 mbar) to obtain a concentrated liquor. The two concentrated liquors were combined and then crystallized at 4-5°C yielding DFX as white crystal.

Other extractive solvents, such as anisole, methyl isobutyl ketone, dibutyl ether, and toluene, can be used in various loadings in the same method (Table 5).

**Table 5. DFX yield and xylose conversion using various heterogenous catalysts and reaction time using the example 5**

| Catalyst | Temp. (°C) | Extractive solvent | React ion time (h) | DFX yield in water (%) | DFX yield in extractive layer (%) | Xylose conversio n (%) |
|---|---|---|---|---|---|---|
| H-form Zeolite Y (SiO₂:Al₂O₃ =80:1) | 140 | Anisole | 4 | 2.2 | 3 | 61 |
| H-form Zeolite Y (SiO₂:Al₂O₃ =80:1) | 140 | MIBK | 4 | 18 | 31 | 86 |
| H-form Zeolite Y (SiO₂:Al₂O₃ =80:1) | 140 | Dibutyl ether | 4 | 49 | 7.7 | 89 |
| H-form Zeolite Y (SiO₂:Al₂O₃ =80:1) | 140 | Toluene | 4 | 31 | 32 | 86 |
| H-form Zeolite Y (SiO₂:Al₂O₃ =80:1) | 140 | CPME | 4 | 12 | 59 | 99 |
| Amberlyst 36 | 140 | CPME | 2 | 17 | 45 | 99 |
| H-form Zeolite Y (SiO₂:Al₂O₃ =80:1) | 150 | CPME | 2 | 19 | 47 | 97 |
| H-form Zeolite Y (SiO₂:Al₂O₃ =80:1) | 100 | CPME | 24 | 12 | 23 | 74 |

### Example 6:

D-xylose (0.25g, 1.67 mmol, 1.0 equiv.), dodecanal (0.75 ml, 2 equiv.) and Y-type zeolite (SiO₂:Al₂O₃=30:1, 0.1 g) were added to dioxane (5 mL) in a 10 mL glass reactor. The mixture was then heated to 65 °C for 5 h with stirring. The resulting solution was cooled to room temperature (~23-25°C), filtered with a nylon membrane filter, and dioxane was removed by rotary evaporator at 45°C water bath. GC-FID measurement showed 66.2% MDX (3,5-*O*-dodecylidene-xylose) yield, 7.2% MDX (1,2-*O*-dodecylidene-xylose) yield, and 3.9% DDX (didodecylidene-xylose) from D-xylose. Similar reactions with other heterogeneous catalysts are summarized in the following table (MDX and DDX yields and xylose conversion using various heterogenous catalysts in the example 6).

| Catalyst | Temperature (°C) | Reaction time (h) | MDX (3,5-*O-*acetal) yield (%) | MDX (1,2-*O-*acetal) yield (%) | DDX yield (%) |
|---|---|---|---|---|---|
| H-form Zeolite β (SiO₂:Al₂O₃=25:1) | 65 | 5 | 42.9 | 3.4 | 3.3 |
| H-form Zeolite β (SiO₂:Al₂O₃=30:1) | 65 | 5 | 45.3 | 2.7 | 10.1 |
| H-form Zeolite β (SiO₂:Al₂O₃=38:1) | 65 | 5 | 44.1 | 2.5 | 7.8 |
| H-form Zeolite β (SiO₂:Al₂O₃=150:1) | 65 | 5 | 42.5 | 2.3 | 24.9 |
| H-form Zeolite Y (SiO₂:Al₂O₃=5.2:1) | 65 | 5 | 42.0 | 10.7 | 1.6 |
| H-form Zeolite Y (SiO₂:Al₂O₃=30:1) | 65 | 5 | 66.2 | 7.2 | 3.9 |
| H-form Zeolite Y (SiO₂:Al₂O₃=60:1) | 65 | 5 | 45.4 | 3.9 | 7.5 |
| H-form Zeolite Y (SiO₂:Al₂O₃=80:1) | 65 | 5 | 50.6 | 3.4 | 9.2 |

### Example 7:

Glyoxylic acid monohydrate (0.29g, 2 equiv.) was added to 1,4-dioxane (5mL). The mixture was pre-dried with 0.6g 4A molecular sieve to remove water. MDX (0.5g, 1.6 mmol, 1 equiv. separated from example 6) was added to the dried mixture with Amberlyst 15 (0.125g) in a 10mL glass reactor. The mixture was then heated to 80 °C for 4 h with stirring. The resulting solution was cooled to room temperature (~23-25°C), filtered with a nylon membrane filter, and dioxane was removed by rotary evaporator at 45°C water bath. GC-FID measurement showed 59.4% GMAX (1,2-O-glyoxylic acid-3,5-O-dodecylidene-xylose) yield.

### Example 8:

D-xylose (5.0 g, 33 mmol, 1.0 equiv.), glyoxylic acid monohydrate (7.66 g, 2.5 equiv.) and a sulfonated resin (Dowex r 50wx8, hydrogen form, 200-400 mesh, 1.5 g) were added to Sulfolane (20 mL) in a 100 mL round bottom flask. The mixture was heated to 90 °C under reduced pressure (40 mbar) for 5 h with stirring by flask rotation. The resulted solution was cooled to room temperature (~23-25°C), filtered with a nylon membrane filter to remove the Dowex catalyst. The yield of diglyoxylic acid xylose (DGAX) was 77% based on the xylose loading as measured by HPLC.

### Example 9:

D-xylose (0.25g, 1.67 mmol, 1.0 equiv.), various aldehydes (2 equiv.) and Y-type zeolite (SiO₂:Al₂O₃=80:1, 0.25 g) were added to 1,4-dioxane (5 mL) in a 10 mL glass reactor. The mixture was then heated to 65 °C for 5h with stirring. The resulting solution was cooled to room temperature (~23-25 °C), filtered with a nylon membrane filter, and dioxane was removed by rotary evaporator at 45 °C water bath.

In comparison, D-xylose (0.25g, 1.67 mmol, 1.0 equiv.), various aldehydes (2 equiv.) and H₂SO₄ (5.3µL) were added to dioxane (5 mL) in a 10 mL glass reactor. The mixture was then heated to 65 °C for 5h with stirring. The resulting solution was cooled to room temperature (~23-25 °C), filtered with a nylon membrane filter, and dioxane was removed by rotary evaporator at 45 °C water bath.

The yields of various products measured by GC-FID from the heterogeneous and homogeneous reactions for each aldehyde type are compared in pairs in the following table (Yields of products IVa, Va, Xa, XIa and I and xylose conversion using various aldehydes in the example 9).

| Aldehyde type | Catalyst | Xylose conversion [%] | Yield of (IVa) [%] | Yield of (Va) [%] | Yield of (Xa) [%] | Yield of (XIa) [%] | Yield of (I) [%] |
|---|---|---|---|---|---|---|---|
| Formaldehyde | Y-type zeolite | 92.5 | 17.7 | 4.1 | 14.7 | 20.8 | 34.4 |
| | H₂SO₄ | 94.8 | 7.9 | 2.3 | 55.7 | 25.1 | 2.6 |
| Propionaldehyde | Y-type zeolite | 84.5 | 27.9 | 0.3 | - | - | 55.0 |
| | H₂SO₄ | 85.8 | 42.3 | 0.3 | - | - | 16.9 |
| Valeraldehyde | Y-type zeolite | 87.9 | 12.1 | 1.4 | - | - | 57.4 |
| | H₂SO₄ | 87.3 | 18.3 | 0.4 | - | - | 21.4 |
| Pivalaldehyde | Y-type zeolite | 82.9 | 18.0 | 18.1 | - | - | 45.7 |
| | H₂SO₄ | 81.1 | 9.9 | 10.6 | - | - | 10.5 |
| Octanal | Y-type zeolite | 89.6 | 50.3 | 1.1 | - | - | 37.3 |
| | H₂SO₄ | 89.4 | 39.2 | 0.4 | - | - | 24.1 |
| Decanal | Y-type zeolite | 88.4 | 58.8 | 2.0 | - | - | 25.8 |
| | H₂SO₄ | 91.2 | 24.493 63 | 0.9237 98 | - | - | 47.8 |
| Dodecanal | Y-type zeolite | 87.6 | 66.1 | 6.27 | - | - | 14.2 |
| | H₂SO₄ | 91.3 | 28.46 | 1.57 | - | - | 28.8 |
| Octodecanal | Y-type zeolite | 57.4 | 38.294 78 | 5.5488 82 | - | - | 2.0 |
| | H₂SO₄ | 87.0 | 26.305 66 | 1.7695 18 | - | - | 18.7 |

### Example 10:

L-arabinose (0.25g, 1.67 mmol, 1.0 equiv.), dodecanal (0.75 ml, 2 equiv.) and Y-type zeolite (SiO₂:Al₂O₃=30:1, 0.25 g) were added to 1,4-dioxane (5 mL) in a 10 mL glass reactor. The mixture was then heated to 65 °C for 5 h with stirring. The resulting solution was cooled to room temperature (~23-25°C), filtered with a nylon membrane filter, and 1,4-dioxane was removed by rotary evaporator at 45°C water bath. GC-FID measurement showed 77.3% partially protected arabinose and 12.7% fully protected arabinose.

### Example 11:

D-glucose (0.3g, 1.67 mmol, 1.0 equiv.), dodecanal (1.85 ml, 5 equiv.) and Y-type zeolite (SiO₂:Al₂O₃=30:1, 0.25 g) were added to 1,4-dioxane (5 mL) in a 10 mL glass reactor. The mixture was then heated to 80 °C for 5 h with stirring. The resulting solution was cooled to room temperature (~23-25°C), filtered with a nylon membrane filter, and 1,4-dioxane was removed by rotary evaporator at 45°C water bath. GC-FID measurement showed 58.0% partially protected glucose yield and 34.3% fully protected glucose.

## Claims

1. Method for preparing an at least partially acetal-protected sugar involving the step of reacting a sugar or a sugar derivative selected from the group consisting of an aldopentose, an aldohexose, an aldopentoside and an aldohexoside with an aldehyde or an aldehyde source wherein the aldehyde source is a polymeric or oligomeric compound that can generate an aldehyde in the reaction conditions, in the presence of heterogeneous acidic catalyst to form the at least partially acetal-protected sugar selected from the group consisting of a compound of formula I, II, III, IV, V, VI, VII, VIII, IX, X, XI and XII 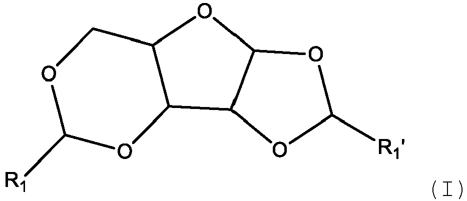 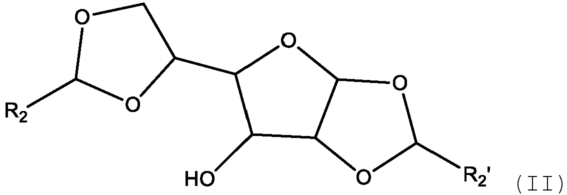 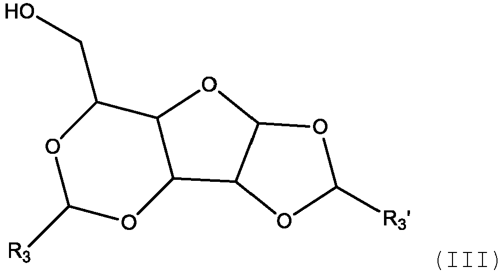 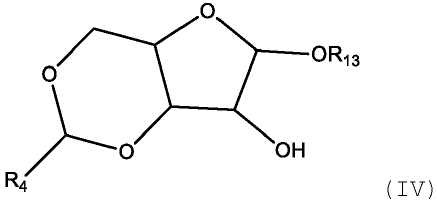 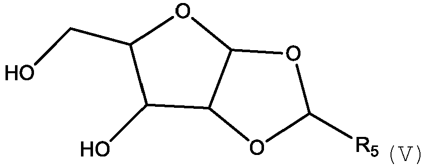 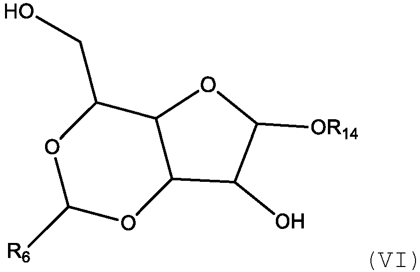 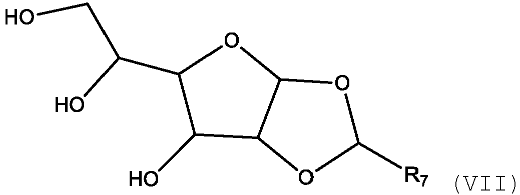 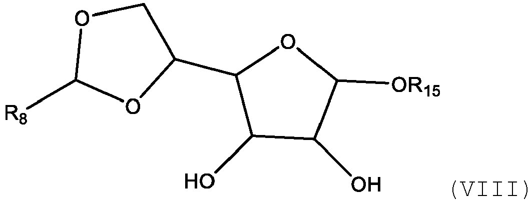 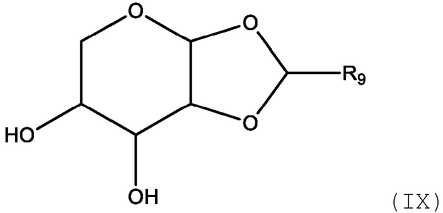 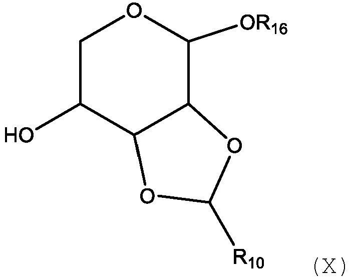 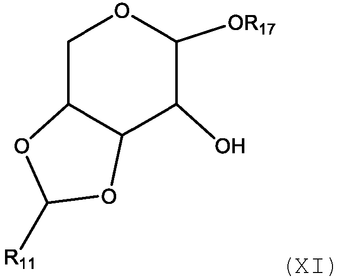 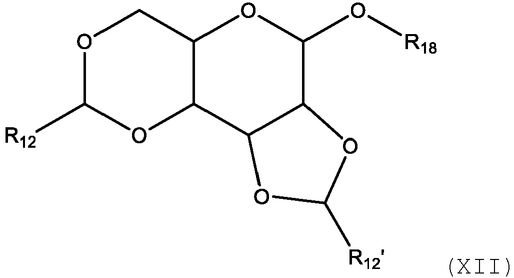
wherein R₁, R₁', R₂, R₂', R₃, R₃', R₄, R₅, R₆, R₇, R₈, R₉, R₁₀, R₁₁, R₁₂ , and R₁₂' are Y or Z-E, and wherein R₁ and R₁', R₂ and R₂', R₃ and R₃', and R₁₂ and R₁₂' are the same or different from each other and
Y is hydrogen or a linear, branched or cyclic hydrocarbon moiety having 1 to 20 carbon atoms,
Z is a linear, branched or cyclic hydrocarbon moiety with 0 to 12 carbon atoms, optionally substituted with 1 to 4 C₁ to C₄ alkyl groups, 1 to 4 halogen atoms, or benzyl groups and
E is -COOH, -CH(COOH)₂, -COOR₁₉, -CH(COOR₂₀)(COOR₂₁), -CHO, - CH(CHO)₂, -C₂H₃, CH(C₂H₃)₂, -CHCHR₂₂ , -CHCR₂₃R₂₄, -C₂H, -C₂R₂₅, -N₃, -NH₂, -CH(NH₂)₂, -NHR₂₆, -CH(NHR₂₇) (NHR₂₈), -NR₂₉R₃₀, - CH(NR₃₁R₃₂)(NR₃₃R₃₄), -OH, -OR₃₅, -CH(R₃₆OH)(R₃₇OH), and
R₁₉, R₂₀, R₂₁, R₂₂, R₂₃, R₂₄, R₂₅, R₂₆, R₂₇, R₂₈, R₂₉, R₃₀, R₃₁, R₃₂, R₃₃ R₃₄, and R₃₅, are independent from each other C₁ to C₂₀ alkyl, and
R₂₀ and R₂₁, R₂₃ and R₂₄, R₂₇ and R₂₈, R₂₉ and R₃₀, R₃₁ and R₃₂, as well as R₃₃ and R₃₄ are the same or different from each other, and
R₃₆ and R₃₇ are independent from each other absent or a linear or branched C₁ to C₁₂ hydrocarbon chain and
R₁₃, R₁₄, R₁₅, R₁₆, R₁₇ and R₁₈ are independent from each other hydrogen or a linear, branched or cyclic hydrocarbon moiety having 1 to 20 carbon atoms.

2. Method according to claim 1, wherein the heterogeneous acidic catalyst is a Brønsted acidic catalyst, preferably selected from the group consisting of
a. acidic zeolite,
b. acidic doped zeolite,
c. acid site-functionalized resin,
d. acid site-functionalized oxide,
e. acidic oxide,
f. heteropolyacids and their derivates.

3. Method according to claim 2, wherein the heterogeneous Brønsted acidic catalyst is an acidic zeolite, preferably comprising
- at least two, preferably two or three, non-interconnected and parallel channel systems wherein, at least one of said channel systems comprises 8- or more-membered ring channels; and a framework Si/X₂ ratio of at least 4 as measured by NMR; or
- at least two, preferably two or three, interconnected and non-parallel channel systems wherein, at least one of said channel systems comprises 10- or more-membered ring channels; and a framework Si/X₂ ratio of at least 4 as measured by NMR; or
- three interconnected and non-parallel channel systems wherein at least two of the channel systems comprise 10-or more-membered ring channels, and a framework Si/X₂ ratio of at least 4 as measured by NMR
wherein each X is Al or B.

4. Method according to any of the preceding claims, wherein the aldehyde is selected from the group consisting of formaldehyde, acetaldehyde, propionaldehyde, butyraldehyde, isobutyraldehyde, valeraldehyde, isovaleraldehyde, hexanal, heptanal, octanal, nonanal, decanal, dodecanal, tetradecanal, hexadecanal, octadecanal, crotonaldehyde, glyoxal, malonic dialdehyde, succinic dialdehyde, glutaraldehyde, adipic dialdehyde, 2-hydroxyadipic dialdehyde, pimelic dialdehyde, suberic dialdehyde, azelaic dialdehyde, sebacic dialdehyde, maleic aldehyde, fumaric aldehyde, phthalaldehyde, isophthalaldehyde, terephthalaldehyde, and 1,4- diformylcyciohexane, glyoxylic acid, glyoxylic acid monohydrate, formyl acetic acid and succinaldehydic acid, preferably formaldehyde, acetaldehyde, dodecanal, glyoxylic acid, glyoxylic acid monohydrate and glutaraldehyde.

5. Method according to any of claims 1 to 3, wherein the aldehyde source is selected from the group consisting of paraformaldehyde, 1,3,5-trioxane, polyoxymethylene and metaldehyde.

6. Method according to any of the preceding claims, wherein R₁₃, R₁₄, R₁₅, R₁₆, R₁₇ and R₁₈ are hydrogen, methyl or ethyl, preferably hydrogen.

7. Method according to any of the preceding claims, the sugar is an aldopentose.

8. Method according to any of the preceding claims, wherein the sugar is arabinose or xylose, preferably D-xylose.

9. Method according to any of claims 1 to 6, wherein the sugar is glucose, preferably D-glucose.

10. Method according to any of the preceding claims, wherein the catalyst has a pore structure.

11. Method according to any of the preceding claims, wherein the reaction is carried out in an organic solvent, preferably selected from the group consisting of dimethyl isosorbide, cyclic ethers, in particular 1,4-dioxane, 2-methyltetrahydrofuran, tetrahydrofuran, sulfolane, sulfolene, aliphatic acids , in particular acetic acid, alkylpyrrolidones, cyclic carbonates, cyclic esters, in particular γ-valerolactone, γ-butyrolactone, acetonitrile, dialkylethers, in particular diethylether, cyclic ethers, in particular CPME and diethylether, cyclic ethers, and glycol monoethers and glycoldiethers.

12. Method according to any of claims 1 to 10, wherein the reaction is carried out in an aqueous solution.

13. Method according to any of claims 1 to 10, wherein the reaction is carried out in a biphasic solvent system, preferably selected from the group consisting of CPME/water, anisole/water, dialkylethers/water, dialkyl ketone/water and toluene/water, preferably CPME/water and toluene/water, and most preferably CPME/water.

14. Method according to any of the preceding claims, wherein the reaction is carried out at a temperature of 50 to 160°C, preferably 80 to 140°C.

## Patentansprüche

1. Verfahren zur Herstellung eines zumindest teilweise acetalgeschützten Zuckers, umfassend den Schritt der Umsetzung eines Zuckers oder eines Zuckerderivats, ausgewählt aus der Gruppe bestehend aus einer Aldopentose, einer Aldohexose, einem Aldopentosid und einem Aldohexosid, mit einem Aldehyd oder einer Aldehydquelle, wobei die Aldehydquelle eine polymerische oder oligomerische Verbindung ist die unter Reaktionsbedingungen einen Aldehyd bilden kann, in Gegenwart eines heterogenen sauren Katalysators, um den zumindest teilweise acetalgeschützten Zucker zu bilden, ausgewählt aus der Gruppe bestehend aus einer Verbindung der Formel I, II, III, IV, V, VI, VII, VIII, IX, X, XI und XII 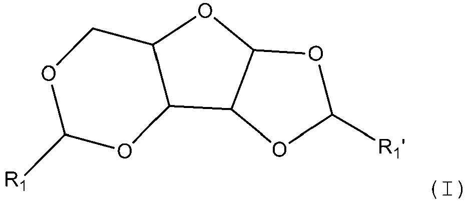 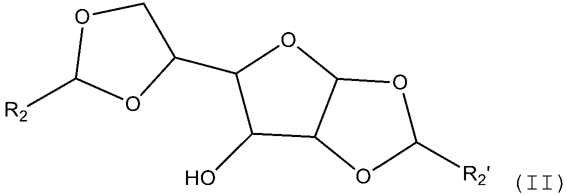 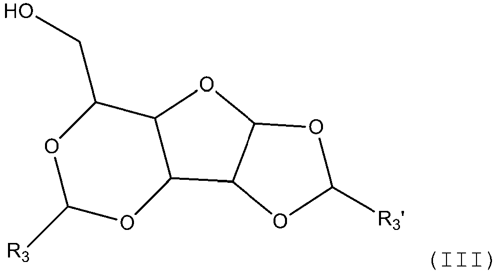 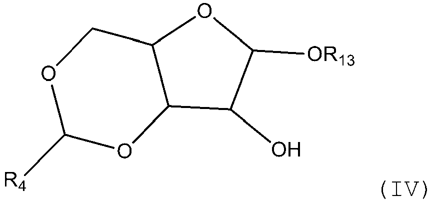 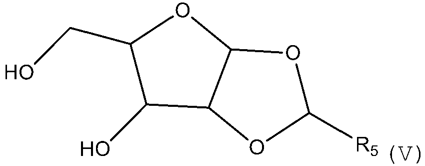 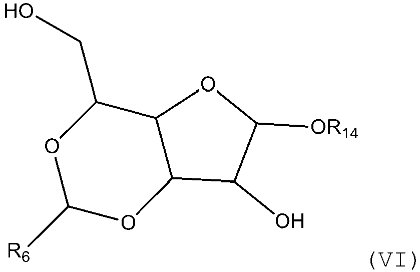 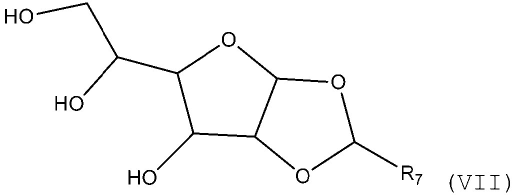 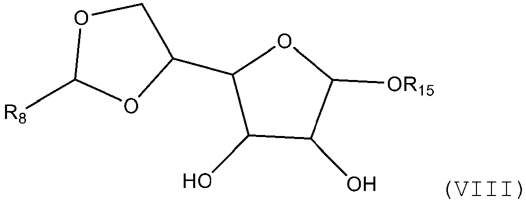 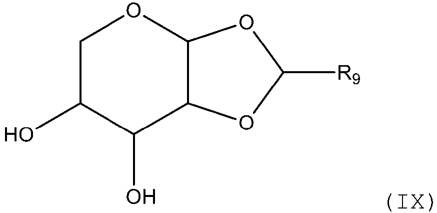 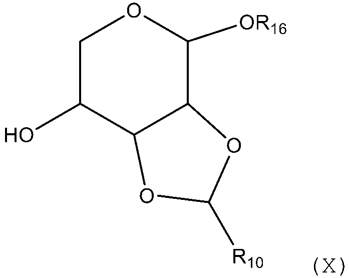 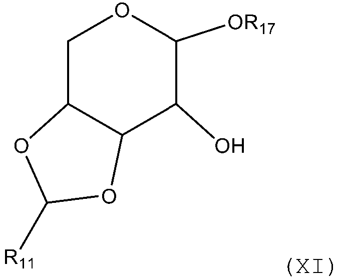 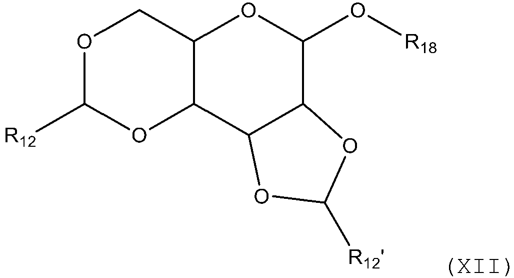
wobei R₁, R₁', R₂, R₂', R₃, R₃', R₄, R₅, R₆, R₇, R₈, R₉, R₁₀, R₁₁, R₁₂ und R₁₂' Y oder Z-E sind, und wobei R₁ und R₁', R₂ und R₂', R₃ und R₃' sowie R₁₂ und R₁₂ ' gleich oder voneinander verschieden sind und Y Wasserstoff oder eine lineare, verzweigte oder cyclische Kohlenwasserstoffgruppe mit 1 bis 20 Kohlenstoffatomen ist,
Z eine lineare, verzweigte oder cyclische Kohlenwasserstoffgruppe mit 0 bis 12 Kohlenstoffatomen ist, die gegebenenfalls mit 1 bis 4 C₁ bis C₄-Alkylgruppen, 1 bis 4 Halogenatomen oder Benzylgruppen substituiert ist, und
E ist -COOH, -CH(COOH) ₂, -COOR₁₉, -CH(COOR₂₀)(COOR₂₁), -CHO, - CH(CHO)₂, -C₂H₃, CH(C₂H₃)₂, -CHCHR₂₂ , -CHCR₂₃R₂₄, -C₂H, -C₂R₂₅, - N₃, -NH₂, -CH(NH₂)₂, -NHR₂₆, -CH(NHR₂₇) (NHR₂₈), -NR₂₉ R₃₀ , -CH(NR₃₁ R₃₂ ) (NR₃₃R₃₄, -OH, -OR₃₅ , -CH(R₃₆OH)(R₃₇OH) und
R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄ und R₃₅ voneinander unabhängige C₁- bis C₂₀-Alkylreste sind und R₂₀ und R₂₁, R₂₃ und R₂₄, R₂₇ und R₂₈, R₂₉ und R₃₀, R₃₁ und R₃₂ sowie R₃₃ und R₃₄ sind gleich oder voneinander verschieden, und
R₃₆ und R₃₇ sind unabhängig voneinander und können entweder nicht vorhanden sein oder eine lineare oder verzweigte C₁ bis C₁₂ - Kohlenwasserstoffkette darstellen, und
R₁₃, R₁₄), R₁₅, R₁₆, R₁₇ und R₁₈ unabhängig voneinander Wasserstoff oder eine lineare, verzweigte oder cyclische Kohlenwasserstoffgruppe mit 1 bis 20 Kohlenstoffatomen darstellen.

2. Verfahren nach Anspruch 1, wobei der heterogene saure Katalysator ein Brønsted-Säure-Katalysator ist, vorzugsweise ausgewählt aus der Gruppe bestehend aus
a. saurem Zeolith,
b. saurem dotiertem Zeolith,
c. mit sauren Stellen funktionalisiertem Harz,
d. mit sauren Stellen funktionalisiertem Oxid,
e. saurem Oxid,
f. Heteropolysäuren und deren Derivate.

3. Verfahren nach Anspruch 2, wobei der heterogene Brønsted-SäureKatalysator ein saurer Zeolith ist, der vorzugsweise enthalten ist
- mindestens zwei, vorzugsweise zwei oder drei, nicht miteinander verbundene und parallele Kanalsysteme, wobei mindestens eines der Kanalsysteme 8- oder mehrgliedrige Ringkanäle enthält; und ein Rahmen-Si/X₂-Verhältnis von mindestens 4, gemessen mittels NMR; oder
- mindestens zwei, vorzugsweise zwei oder drei, miteinander verbundene und nicht-parallele Kanalsysteme, wobei mindestens eines der Kanalsysteme 10- oder mehrgliedrige Ringkanäle enthält; und ein Si/X₂-Verhältnis des Gerüsts von mindestens 4, gemessen mittels NMR; oder
- drei miteinander verbundene und nicht parallele Kanalsysteme, wobei mindestens zwei der Kanalsysteme 10- oder mehrgliedrige Ringkanäle enthalten, und ein mittels NMR gemessenes Si/X₂-Verhältnis des Gerüsts von mindestens 4
wobei jedes X für Al oder B steht.

4. Verfahren nach einem der vorhergehenden Ansprüche, wobei der Aldehyd aus der Gruppe ausgewählt ist, bestehend aus Formaldehyd, Acetaldehyd, Propionaldehyd, Butyraldehyd, Isobutyraldehyd, Valeraldehyd, Isovaleraldehyd, Hexanal, Heptanal, Octanal, Nonanal, Decanal, Dodecanal, Tetradecanal, Hexadecanal, Octadecanal, Crotonaldehyd, Glyoxal, Malondialdehyd, Bernsteindialdehyd, Glutaraldehyd, Adipindialdehyd, 2-Hydroxyadipindialdehyd, Pimelindialdehyd, Suberdialdehyd, Azelaindialdehyd, Sebacindialdehyd, Maleinaldehyd, Fumarsäurealdehyd, Phthalsäurealdehyd, Isophthalaldehyd, Terephthalaldehyd und 1,4-Diformylcyclohexan, Glyoxylsäure, Glyoxylsäure-Monohydrat, Formylessigsäure und Succinaldehydsäure, vorzugsweise Formaldehyd, Acetaldehyd, Dodecanal, Glyoxylsäure, Glyoxylsäure-Monohydrat und Glutaraldehyd.

5. Verfahren nach einem der Ansprüche 1 bis 3, wobei die Aldehydquelle aus der Gruppe ausgewählt ist, die aus Paraformaldehyd, 1,3,5-Trioxan, Polyoxymethylen und Metaldehyd besteht.

6. Verfahren nach einem der vorhergehenden Ansprüche, wobei R₁₃, R₁₄, R₁₅, R₁₆, R₁₇ und R₁₈ Wasserstoff, Methyl oder Ethyl, vorzugsweise Wasserstoff, sind.

7. Verfahren nach einem der vorhergehenden Ansprüche, wobei der Zucker eine Aldopentose ist.

8. Verfahren nach einem der vorhergehenden Ansprüche, wobei der Zucker Arabinose oder Xylose, vorzugsweise D-Xylose, ist.

9. Verfahren nach einem der Ansprüche 1 bis 6, wobei der Zucker Glucose, vorzugsweise D-Glucose, ist.

10. Verfahren nach einem der vorhergehenden Ansprüche, wobei der Katalysator eine Porenstruktur aufweist.

11. Verfahren nach einem der vorhergehenden Ansprüche, wobei die Reaktion in einem organischen Lösungsmittel durchgeführt wird, das vorzugsweise aus der Gruppe ausgewählt ist, bestehend aus Dimethylisosorbid, cyclischen Ethern, insbesondere 1,4-Dioxan, 2-Methyltetrahydrofuran, Tetrahydrofuran, Sulfolan, Sulfolen, aliphatischen Säuren, insbesondere Essigsäure, Alkylpyrrolidone, cyclische Carbonate, cyclische Ester, insbesondere γ-Valerolacton, γ-Butyrolacton, Acetonitril, Dialkylether, insbesondere Diethylether, cyclische Ether, insbesondere CPME und Diethylether, sowie Glykolmonoether und Glykoldiether.

12. Verfahren nach einem der Ansprüche 1 bis 10, wobei die Reaktion in einer wässrigen Lösung durchgeführt wird.

13. Verfahren nach einem der Ansprüche 1 bis 10, wobei die Reaktion in einem zweiphasigen Lösungsmittelsystem durchgeführt wird, das vorzugsweise aus der Gruppe ausgewählt ist, die aus CPME/Wasser, Anisol/Wasser, Dialkylethern/Wasser, Dialkylketon/Wasser und Toluol/Wasser besteht, vorzugsweise aus CPME/Wasser und Toluol/Wasser und am bevorzugtesten aus CPME/Wasser.

14. Verfahren nach einem der vorhergehenden Ansprüche, wobei die Reaktion bei einer Temperatur von 50 bis 160 °C, vorzugsweise 80 bis 140 °C, durchgeführt wird.

## Revendications

1. Procédé de préparation d'un sucre au moins partiellement protégé par un acétal, comprenant l'étape consistant à faire réagir un sucre ou un dérivé de sucre choisi dans le groupe constitué par un aldopentose, un aldohexose, un aldopentoside et un aldohexoside avec un aldéhyde ou une source d'aldéhyde, ladite source d'aldéhyde étant un composé polymère ou oligomère capable de former un aldéhyde dans les conditions de la réaction, en présence d'un catalyseur acide hétérogène pour former le sucre au moins partiellement protégé par un acétal choisi dans le groupe constitué par un composé de formule I, II, III, IV, V, VI, VII, VIII, IX, X, XI et XII 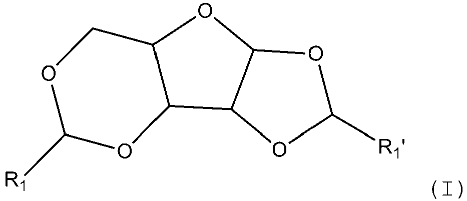 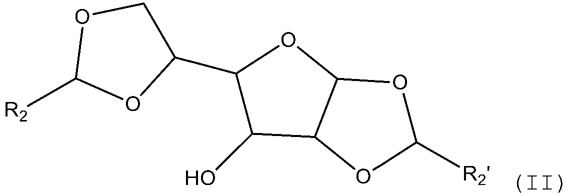 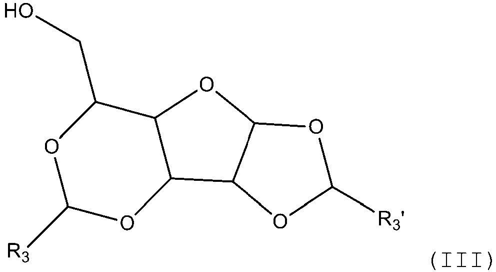 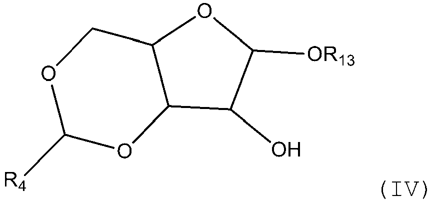 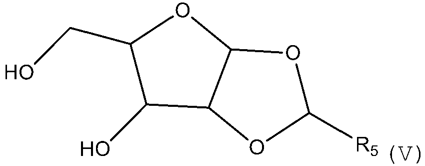 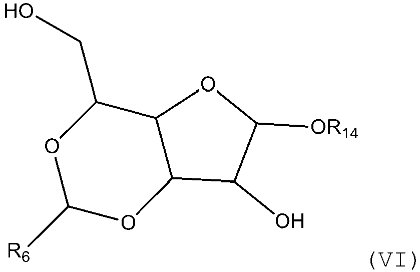 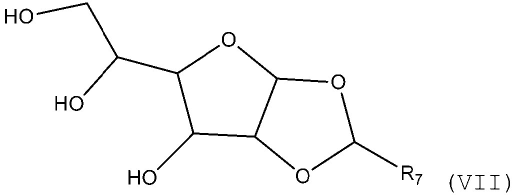 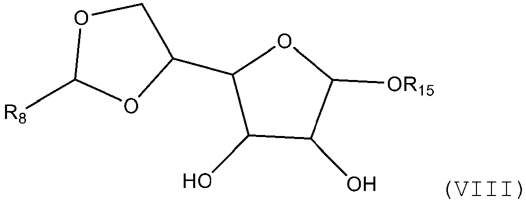 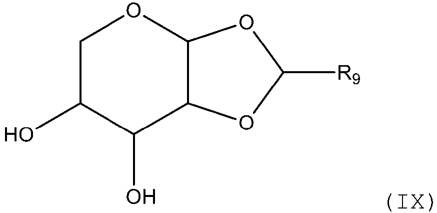 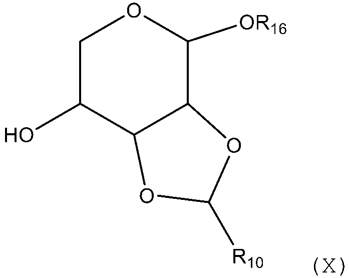 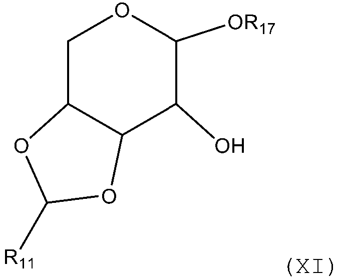 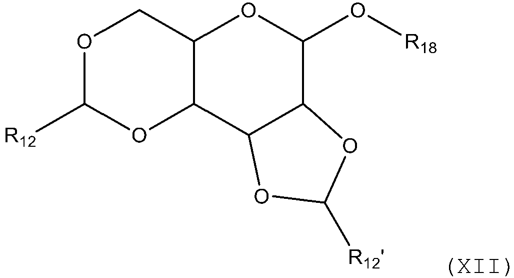
où R₁, R₁', R₂, R₂', R₃, R₃', R₄, R₅, R₆, R₇, R₈, R₉, R₁₀, R₁₁, R₁₂ et R₁₂' représentent Y ou Z-E, et dans laquelle R₁et R₁', R₂ et R₂', R₃ et R₃', ainsi que R₁₂ et R₁₂' sont identiques ou différents les uns des autres et
Y représente un atome d'hydrogène ou un groupement hydrocarboné linéaire, ramifié ou cyclique comportant de 1 à 20 atomes de carbone,
Z est un groupement hydrocarboné linéaire, ramifié ou cyclique comportant de 0 à 12 atomes de carbone, éventuellement substitué par 1 à 4 groupes alkyle en C₁à C₄, 1 à 4 atomes d'halogène ou des groupes benzyle, et
E est -COOH, -CH(COOH)₂, -COOR₁₉, -CH(COOR₂₀)(COOR₂₁), -CHO, - CH(CHO)₂, -C₂H₃, CH(C₂H₃)₂, -CHCHR₂₂ , -CHCR₂₃R₂₄, -C₂H, -C₂R₂₅, -N₃ , -NH₂ , -CH(NH₂)₂ , -NHR₂₆ , -CH(NHR₂₇)(NHR₂₈), -NR₂₉R₃₀ , -CH(NR₃₁ R₃₂)(NR₃₃R₃₄), -OH, -OR₃₅ , -CH(R₃₆OH)(R₃₇OH), et
R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄et R₃₅ représentent, indépendamment les uns des autres, des groupes alkyleₑₙ₂₀ C₁à C, et
R₂₀ et R₂₁, R₂₃ et R₂₄, R₂₇ et R₂₈, R₂₉ et R₃₀, R₃₁ et R₃₂, ainsi que R₃₃ et R₃₄ sont identiques ou différents les uns des autres, et
R₃₆ et R₃₇ sont indépendants l'un de l'autre, absents ou représentant une chaîne hydrocarbonée linéaire ou ramifiée en C₁ à C₁₂ ,et
R₁₃, R₁₄, R₁₅, R₁₆, R₁₇ et R₁₈ sont indépendants les uns des autres et représentent un atome d'hydrogène ou un groupement hydrocarboné linéaire, ramifié ou cyclique comportant de 1 à 20 atomes de carbone.

2. Procédé selon la revendication 1, dans lequel le catalyseur acide hétérogène est un catalyseur acide de Brønsted, choisi de préférence parmi le groupe constitué de
a. une zéolite acide,
b. une zéolite dopée acide,
c. une résine fonctionnalisée par des sites acides,
d. un oxyde fonctionnalisé par des sites acides,
e. un oxyde acide,
f. des hétéropolyacides et leurs dérivés.

3. Procédé selon la revendication 2, dans lequel le catalyseur acide de Brønsted hétérogène est une zéolite acide, comprenant de préférence
- au moins deux, de préférence deux ou trois, systèmes de canaux parallèles et non interconnectés, dans lesquels au moins l'un desdits systèmes de canaux comprend des canaux à cycles à 8 membres ou plus ; et un rapport Si/X₂du réseau d'au moins 4, tel que mesuré par RMN ; ou
- au moins deux, de préférence deux ou trois, systèmes de canaux interconnectés et non parallèles, dans lesquels au moins l'un desdits systèmes de canaux comprend des canaux à cycles à 10 membres ou plus ; et un rapport Si/X₂du réseau d'au moins 4, tel que mesuré par RMN ; ou
- trois systèmes de canaux interconnectés et non parallèles, dans lesquels au moins deux des systèmes de canaux comprennent des canaux cycliques à 10 membres ou plus, et un rapport Si/X₂du squelette d'au moins 4, tel que mesuré par RMN
où chaque X représente Al ou B.

4. Procédé selon l'une quelconque des revendications précédentes, dans lequel l'aldéhyde est choisi parmi le groupe constitué du formaldéhyde, de l'acétaldéhyde, du propionaldéhyde, du butyraldéhyde, de l'isobutyraldéhyde, du valéraldéhyde, de l'isovaléraldéhyde, de l'hexanal, de l'heptanal, de l'octanal, du nonanal, du décanal, du dodécanal, du tétradécanal, de l'hexadécanal, de l'octadécanal, du crotonaldéhyde, le glyoxal, le dialdéhyde malonique, le dialdéhyde succinique, le glutaraldéhyde, le dialdéhyde adipique, le dialdéhyde 2-hydroxyadipique, le dialdéhyde pimélique, le dialdéhyde subérique, le dialdéhyde azélaïque, le dialdéhyde sébacique, l'aldéhyde maléique, l'aldéhyde fumarique, le phtalaldéhyde, isophtalaldéhyde, le téréphtalaldéhyde et le 1,4-diformylcyclohexane, l'acide glyoxylique, l'acide glyoxylique monohydraté, l'acide formylacétique et l'acide succinaldéhydique, de préférence le formaldéhyde, l'acétaldéhyde, le dodécanal, l'acide glyoxylique, l'acide glyoxylique monohydraté et le glutaraldéhyde.

5. Procédé selon l'une quelconque des revendications 1 à 3, dans lequel la source d'aldéhyde est choisie parmi le groupe constitué du paraformaldéhyde, du 1,3,5-trioxane, du polyoxyméthylène et du métaldéhyde.

6. Procédé selon l'une quelconque des revendications précédentes, dans lequel R₁₃, R₁₄, R₁₅, R₁₆, R₁₇ et R₁₈ représentent un atome d'hydrogène, un groupe méthyle ou un groupe éthyle, de préférence un atome d'hydrogène.

7. Procédé selon l'une quelconque des revendications précédentes, dans lequel le sucre est un aldopentose.

8. Procédé selon l'une quelconque des revendications précédentes, dans lequel le sucre est l'arabinose ou le xylose, de préférence le D-xylose.

9. Procédé selon l'une quelconque des revendications 1 à 6, dans lequel le sucre est le glucose, de préférence le D-glucose.

10. Procédé selon l'une quelconque des revendications précédentes, dans lequel le catalyseur présente une structure poreuse.

11. Procédé selon l'une quelconque des revendications précédentes, dans lequel la réaction est réalisée dans un solvant organique, de préférence choisi parmi le groupe constitué du diméthylisosorbide, des éthers cycliques, en particulier le 1,4-dioxane, le 2-méthyltétrahydrofurane, le tétrahydrofurane, le sulfolane, le sulfolène, des acides aliphatiques, en particulier l'acide acétique, des alkylpyrrolidones, des carbonates cycliques, des esters cycliques, en particulier la γ-valérolactone, la γ-butyrolactone, l'acétonitrile, des éthers dialkyliques, en particulier l'éther diéthylique, des éthers cycliques, en particulier le CPME et l'éther diéthylique, des éthers cycliques, ainsi que des monoéthers de glycol et des diéthers de glycol.

12. Procédé selon l'une quelconque des revendications 1 à 10, dans lequel la réaction est réalisée dans une solution aqueuse.

13. Procédé selon l'une quelconque des revendications 1 à 10, dans lequel la réaction est réalisée dans un système de solvants biphasique, choisi de préférence parmi le groupe constitué de CPME/eau, d'anisole/eau, d'éthers dialkyliques/eau, de cétones dialkyliques/eau et de toluène/eau, de préférence de CPME/eau etde toluène/eau, et de manière encore plus préférée de CPME/eau.

14. Procédé selon l'une quelconque des revendications précédentes, dans lequel la réaction est réalisée à une température comprise entre 50 et 160 °C, de préférence entre 80 et 140 °C.
